# EUROPEAN PATENT SPECIFICATION

(11) **EP 2 781 907 B1**
(45) Date of publication and mention of the grant of the patent: **26.12.2018**
(21) Application number: 12849361.6
(22) Date of filing: 13.11.2012
(51) Int. Cl.: G01N 21/84, B01L 3/00

(54) **REFLECTIVE ABSORBANCE MEASURING DEVICE, AND INTEGRAL APPARATUS FOR ANALYZING REFLECTIVE ABSORBANCE AND LATERAL FLOW**
MESSVORRICHTUNG FÜR REFLEKTIERENDE ABSORBANZ UND INTEGRIERTE VORRICHTUNG ZUR ANALYSE VON REFLEKTIERENDER ABSORBANZ UND LATERALFLUSS
DISPOSITIF DE MESURE D'ABSORBANCE RÉFLÉCHISSANTE ET APPAREIL D'UN SEUL TENANT POUR ANALYSE D'ABSORBANCE RÉFLÉCHISSANTE ET ÉCOULEMENT LATÉRAL

(30) Priority: 14.11.2011 KR 20110118104
(43) Date of publication of application: 24.09.2014
(73) Proprietor: Boditech Med Inc., Gangwon-do 24398 (KR)
(72) Inventor: KIM, Byeong Chul, Chuncheon-si Gangwon-do 200-160 (KR); LEE, Jae-min, Chuncheon-si Gangwon-do 200-160 (KR); JEONG, Jeong-hyeok, Hongcheon-gun Gangwon-do 250-885 (KR)
(74) Representative: Ter Meer Steinmeister & Partner
(86) International application number: PCT/KR2012/009549
(87) International publication number: WO 2013/073811

(56) References cited:
- WO-A1-2004/097418
- JP-A- 2011 089 917
- KR-A- 20110 127 386
- KR-B1- 100 531 760
- KR-B1- 100 560 174
- US-A1- 2002 019 062
- US-A1- 2005 229 696
- US-B1- 6 235 241

## Description

### TECHNICAL FILED

The present disclosure relates to a device for analyzing biological samples, particularly a lateral flow assay device, and an integrated device for both measuring reflective absorbance and performing lateral flow assay.

### BACKGROUND

Diagnostic methods and devices for qualitatively or quantitatively measuring a finite amount of materials contained in biological samples such as blood or urine have been rapidly developed for the last 30 years. Since a radioimmunoassay (RIA) using radioisotopes was first introduced in the 1950s, other assays such as enzyme-linked immunosorbent assay (ELISA) were also developed in the 1970s and 1980s. Currently, ELISA is one of the most widely used methods, and became an indispensable tool in the field of medical and life sciences. In recent years, a modified ELISA method has been developed, and among them is to use a plurality of antibodies fixed to 96-well plates to analyze a large number of samples at the same time.

In a typical diagnostic method including RIA or ELISA, usually only one type of analyte can be quantitatively measured by using a high-priced device in a laboratory equipped therewith through a complex multi-step process. Thus, the method is not suitable for use in a small-sized hospital, an emergency room, or at home, which is not equipped with the proper facility or equipment.

A diagnostic product developed to solve these shortcomings is a simple diagnostic kit based on the immunochromatography. By using this diagnostic kit, a particular analyte contained in a biological sample such as whole blood, blood serum, and urine can be readily detected quantitatively and/or qualitatively.

However, the immunochromatography which is based on a specific binding between antibody-antigen often needs to be corrected and/or complemented with other tests for accurate results. This in many cases requires additional batch of biological samples to obtain additional test results to such as the concentration of hemoglobin contained in the biological sample which requires the use of a separate absorbance measuring device/device.

The absorbance is generally measured using the light that has passed through a sample after the sample being illuminated with an incidence light of a particular wavelength.

However, this requires a large amount of sample and further the result may not be reliable due to an air layer that may be present in the light pass during the measurement.

Moreover when both assays, i.e., the immunochromatography and the absorbance assays are required, each assay is generally performed in a separate device, first by performing the immunochromatography followed by measuring the absorbance of the transmitted light through the immunochromatography result. But these caused problems in the proper arrangement to obtain accurate result of the components of the devices employed, such as a light source, a light detector, and the device in which a sample is analyzed.

For example, Korean Patent Application Publication No. 10-2007-0002042 provides a test member for measuring a concentration of a material contained in a physiological body fluid.

Korean Patent No. 298130 provides a device and a method for measuring cholesterol.

US 6235241 relates to an assay result reader, for use in conjunction with an assay device comprising a porous liquid-permeable carrier in the form of a strip or sheet through the thickness of which electromagnetic radiation is transmissible, the carrier including a detection zone in which an assay result is revealed by specific binding of a detectable material directly or indirectly to a binding agent immobilized in the detection zone, detection of the detectable material being effected by determining the extent to which electromagnetic radiation transmitted through the thickness of said carrier is attenuated by the presence of the detectable material bound in the detection zone.

US 2005/0229696 relates to an analytical chip which is provided with a flow channel, whose section is in a closed shape and through which a fluid sample (Fs) is made to flow, for carrying out analysis regarding the fluid sample (Fs) based on interaction between a predetermined substance and a specific substance, which is placed facing said flow channel. The chip further has a projection member attached to said flow channel. With this arrangement, it becomes possible to analyze the fluid sample (Fs) efficiently with high precision.

WO 2044/097418 relates to a diagnostic kit.

But no documents disclose a device for measuring a reflective absorbance and an integrated device for measuring absorbance and performing immunochromatography at once. Thus there is a need for improved devices which are convenient to use and produce accurate results with a small amount of sample.

### DETAILED DESCRIPTION OF THE INVENTION

### PROBLEMS TO BE SOLVED

The present disclosure has been made in an effort to solve the above-mentioned problems, and the present disclosure is to provide a lateral flow analyzing device which make possible a fast and convenient measurement without sacrificing the accuracy of the result.

Further the present disclosure is also to provide a device where the device for measuring reflective absorbance and the device for lateral flow assay are integrated into one, which makes possible a fast and a convenient measurement/assay without sacrificing the accuracy.

### SUMMARY OF THE INVENTION

In one aspect of the present disclosure, there is provided a device for measuring reflective absorbance, the device comprising: a base member comprising a sample receiving part; and a cover member covering the base member; the cover member comprising a sample inlet through which a sample is introduced into the sample receiving part and a light transmitting member, the light transmitting member being positioned such that it is located perpendicular to the sample receiving part when the cover member covers the base member, wherein the sample receiving part is configured to reflect an incident light illuminated through the light transmitting member.

According to one embodiment of the present disclosure, the sample receiving part is configured either to directly reflect the incident light or further comprises a reflective bottom portion which it is able to reflect the incident light.

According to other embodiment of the present disclosure, the base member comprises a window into which the light transmitting member is engaged, or the light transmitting member constitutes a part of the cover member and is configured to be integrally formed with the cover member, in which case the cover member is made of a light transparent material and all or part of the surface of the cover member other than the light transmitting member is configured to be light-blocked.

According to still other embodiment of the present disclosure, the light transmitted through the cover member other than the light transmitting member is physically blocked by covering the surface of the corresponding cover member with a light blocking material or the light may be blocked by using appropriate software when the analysis results are interpreted by a reader. Or the cover member, in all or in part may be made of a light blocking material except the light transmitting member.

According to still other embodiment of the present disclosure, the bottom of the light transmitting member is separated from the surface of the sample receiving part about 0.5 mm to 10 mm.

According to still other embodiment of the present disclosure, a tetragonal hole is formed on the cover member, and a tetragonal portion is formed perpendicular to the tetragonal hole on the corresponding area of the base member when the cover member and the base member are engaged.

According to still other embodiment of the present disclosure, the cover member comprises one or more sample inlets, particularly two sample inlets and when the cover member comprises a plurality of sample inlets, the sample inlets being arranged to face each other with the light transmitting member being disposed therebetween.

According to still other embodiment of the present disclosure, the cover member comprise one sample inlet and further comprises an opening, and the opening being positioned to face the sample inlet with the light transmitting member being disposed therebetween.

According to still other embodiment of the present disclosure, the distance underneath the bottom of the left and the right sides of the light transmitting member have an identical or different height or the bottom of the light transmitting member is configured to be recessed inward or concaved.

According to still other embodiment of the present disclosure, the cover member comprises a protrusion formed on the bottom thereof adjacent to the light transmitting member.

According to still other embodiment of the present disclosure, the present device comprises two sample inlets or one sample inlet and one opening, in which case each of the components comprised is positioned facing each other with the light transmitting member being arranged therebetween, and the protrusion is formed on one of the sample inlets or the area where the opening is formed.

In other aspect of the present disclosure, there is provided an integrated device for measuring reflective absorbance comprising two or more devices of the present disclosure as described above.

According to one embodiment of the present disclosure, each device contained in the integrated device is disposed side by side in parallel or in a row longitudinally.

In another aspect of the present disclosure, there is provided a system for measuring reflective absorbance, the system comprising: a device according to the present disclosure as described above; a light source being disposed above the cover member of the device; and a light detector being disposed above the cover member, the detector measuring the amount of light being reflected from the sample receiving part after a light illumination through the light transmitting member from the light source.

According to one embodiment of the present disclosure, more than one the light detectors are used and they are disposed askew on each side of the light source.

In another aspect of the present disclosure, there is provided a device for lateral flow assay according to the claim 1, the device comprising: a base member comprising a strip receiving part; a cover member covering the base member and the cover member comprising a sample inlet and a window; and a window cover for opening and closing the window, wherein a sample is introduced onto a strip mounted on the strip receiving part through the sample inlet and the window cover is positioned being perpendicular to the strip receiving part when the window is closed.

According to one embodiment of the present disclosure, the sample inlet contained in the device for lateral flow assay are configured to be positioned in an area of the cover member adjacent to the sample inlet formed on the cover member of the device for measuring absorbance, the two sample inlets being arranged in a row.

According to one embodiment of the present disclosure, the window cover is removably attached, and the window cover is open or closed by sliding or pulling upward and downward.

In another aspect of the present disclosure, there is provided an integrated device for measuring reflective absorbance and lateral flow assay according to the claim 3.

According to one embodiment of the present disclosure, the present device further comprises a window cover, wherein the cover is detachable, and is able to open or close the window, and is positioned being perpendicular to the strip receiving part in a closed state.

According to still other embodiment of the present disclosure, the integrated device comprises two or more devices for measuring reflective absorbance and two or more device for lateral flow assay, and each of the devices for measuring reflective and the device for lateral flow assay being disposed in a row longitudinally or side by side in parallel.

According to still other embodiment of the present disclosure, the integrated device comprises two devices for measuring reflective absorbance and two devices for lateral flow assay, wherein one from each device is disposed in a row forming a first set and the other from each device is disposed in a row forming a second set of device, and the two set of devices are then arranged side by side.

### ADVANTAGEOUS EFFECTS

The device according to the present disclosure provides convenient loading of a sample and also provides an accurate, reliable and reproducible result.

Further, the integrated device of the present disclosure makes it possible to perform a lateral flow assay and absorption measurement in one device, which provides rapid and accurate analysis and improvement in user convenience without scarifying the accuracy of results. Also the accuracy of result is further improved by the strips used for lateral flow assay which are protected and are only exposed just before use.

### BRIEF DESCRIPTION OF THE DRAWINGS

FIG. 1 is an exploded perspective view of the device for measuring reflective absorbance according to one embodiment of the present disclosure in which the light transmitting member are integrated with the cover member.
FIG. 2 is an exploded perspective view of the device for measuring reflective absorbance according to one embodiment of the present disclosure in which the cover member comprises a window to which the transmitting member is engaged.
FIG. 3 is a perspective view of the device for measuring reflective absorbance according to one embodiment of the present disclosure.
FIG. 4 is a top view of the cover member of the device for measuring reflective absorbance according to one embodiment of the present disclosure.
FIG. 5 is a top view of the base member of the device for measuring reflective absorbance according to one embodiment of the present disclosure.
FIG. 6 is a sectional view taken along line VI-VI of FIG. 3 without (a) or with (b) a sample.
FIG. 7 is a sectional view taken along line VI-VI of FIG. 3 schematically showing one exemplary light paths when using the device for measuring reflective absorbance according to one embodiment of the present disclosure.
FIG. 8 is a side view of the device for measuring reflective absorbance according to one embodiment of the present disclosure.
FIG. 9A is an exploded perspective view of the integrated device for measuring reflective absorbance and performing lateral flow assay according to one embodiment of the present disclosure in which a light transmitting member is formed being integrated with the cover member.
FIG. 9B is an exploded perspective view of the integrated device for measuring reflective absorbance and performing lateral flow assay according to one embodiment of the present disclosure in which the cover member comprises a window to which the light transmitting member is engaged.
FIG. 10 is an exploded perspective view showing the integrated device for measuring reflective absorbance and performing lateral flow assay according to one embodiment of the present disclosure with a strip mounted.
FIG. 11 is a perspective view of the integrated device for measuring reflective absorbance and performing lateral flow assay according to one embodiment of the present disclosure in which the cover member and the base member are engaged to each other closing the device.
FIG. 12 is a top view of the cover member of the integrated device for measuring reflective absorbance and performing lateral flow assay according to one embodiment of the present disclosure.
FIG. 13 is a top view of the base member of the integrated device for measuring reflective absorbance and performing lateral flow assay according to one embodiment of the present disclosure.
FIG. 14 is a sectional view taken along line XIV-XIV of FIG. 11.
FIG. 15 is a side view of the integrated device for measuring reflective absorbance and performing lateral flow assay according to one embodiment of the present disclosure.
FIG. 16A is a perspective view of the device for measuring reflective absorbance according to one embodiment of the present disclosure in which two devices are arranged side by side.
FIG. 16B is a perspective view of the device for measuring reflective absorbance according to one embodiment of the present disclosure in which three devices are arranged in a row from top to bottom.
FIG. 16C is a perspective view of the integrated device for measuring reflective absorbance and performing lateral flow assay according to one embodiment of the present disclosure in which two integrated devices are arranged side by side.
FIG. 16D is a perspective view of the integrated device for measuring reflective absorbance and performing lateral flow assay according to one embodiment of the present disclosure in which two integral devices, each having a transmitting member inserted into a window, are arranged side by side.
FIG. 17 is a graph comparing the Hb concentration obtained by the present device for measuring reflective absorbance or by the device integrating reflective absorbance measurement and lateral flow assay with the Hb concentration measured using a conventional method, wherein the absorbance measured was converted into a concentration of Hb.
FIG. 18 is a graph comparing the HbAlc concentration obtained by the present device for measuring reflective absorbance or by the device integrating reflective absorbance measurement and lateral flow assay with the HbA1c concentration measured using a conventional method, wherein the Hb concentration was determined by the absorbance and A1c was determined by the lateral flow assay.
FIG. 19A is a sectional view taken along line VI-VI of FIG. 3 or line XIV-XIV of FIG. 11 showing the light transmitting member and the components adjacent thereto, i.e., the sample inlets and the bottom of the light transmitting member touching the sample according to one embodiment of the present disclosure.
FIG. 19B is a sectional view taken along line VI-VI of FIG. 3 or line XIV-XIV of FIG. 11 showing the light transmitting member and the components adjacent thereto, i.e., the opening, the sample inlet, and the bottom of the light transmitting member touching the sample according to one embodiment of the present disclosure.
FIG. 19C is a sectional view taken along line VI-VI of FIG. 3 or line XIV-XIV of FIG. 11 showing the light transmitting member and the components adjacent thereto, i.e., the sample inlets and the bottom of the light transmitting member which is recessed inward and contacted with the sample.
FIG. 19D is a sectional view taken along line VI-VI of FIG. 3 or line XIV-XIV of FIG. 11 showing the light transmitting member and the components adjacent thereto, i.e., the opening, the sample inlet, and the bottom of the light transmitting member which is recessed inward and contacted with the sample.
FIG. 20A is a sectional view taken along line VI-VI of FIG. 3 or line XIV-XIV of FIG. 11 showing the light transmitting member and the components adjacent thereto, i.e., the light transmitting member, the sample inlet and the cover member with a protrusion.
FIG. 20B is identical to FIG. 20a, except that an opening is formed on the left side instead of the sample inlet on the left side.
FIG. 20C is a schematic diagram showing that a temporary air pressure built up inside the sample receiving part of the present device due to a sample loading which makes the sample filling from the entire bottom of the sample receiving part and thus generates air bubbles is prevented. Instead the air pressure built up is released through the opening by filling the sample receiving part from a side of the sample inlet.
FIG. 21 is a schematic diagram showing that the window cover closing the window of the device of the present disclosure is opened for measurement in a reader while the device is moving along the moving guide when it is inserted into a reader.
FIG. 22A is a perspective view of the integrated device for measuring reflective absorbance and lateral flow assay according to one embodiment of the present disclosure in which the window cover is opened.
FIG. 22B is a perspective view of the integrated device for measuring reflective absorbance and lateral flow assay according to one embodiment of the present disclosure in which the window cover is closed.
FIG. 23A is a perspective view showing the bottom of the window cover according to one embodiment of the present disclosure.
FIG. 23B is a side view of the window cover of FIG. 23a.
FIG. 24 is a perspective view and a sectional view showing the components and structures related to semiautomatic opening and closing thereof.
FIG. 25 is an exploded perspective view of the integrated device for measuring a reflective absorbance and a lateral flow assay according to one embodiment of the present disclosure comprising a window cover.
FIG. 26 is a perspective view of an integrated device for measuring reflective absorbance and lateral flow assay according to one embodiment of the present disclosure in which the cover member and the base member are engaged to each other.
FIG. 27 is a perspective view of an integrated device for measuring reflective absorbance and lateral flow assay according to one embodiment of the present disclosure in which the cover member comprises a window cover and it is engaged to the base member.
FIG. 28 is a sectional view of an integrated device for measuring a reflective absorbance and a lateral flow assay according to one embodiment of the present disclosure taken along line XV-XV of FIG. 27.
FIG. 29 is an exploded perspective view of the device for lateral flow assay according to one embodiment of the present disclosure including the measurement window cover, the cover member, the strip and the base member.
FIG. 30 is a perspective view of the device for lateral flow assay according to one embodiment of the present disclosure in which the cover member comprises a window cover and it is engaged to the base member.
FIG. 31 is a sectional view of the device for lateral flow assay according to one embodiment of the present disclosure, taken along line XVI-XVI of FIG. 30.

### DETAILED DESCRIPTION OF THE EMBODIMENTS

Hereafter, exemplary embodiments of the present disclosure will be described with reference to the accompanying drawings.

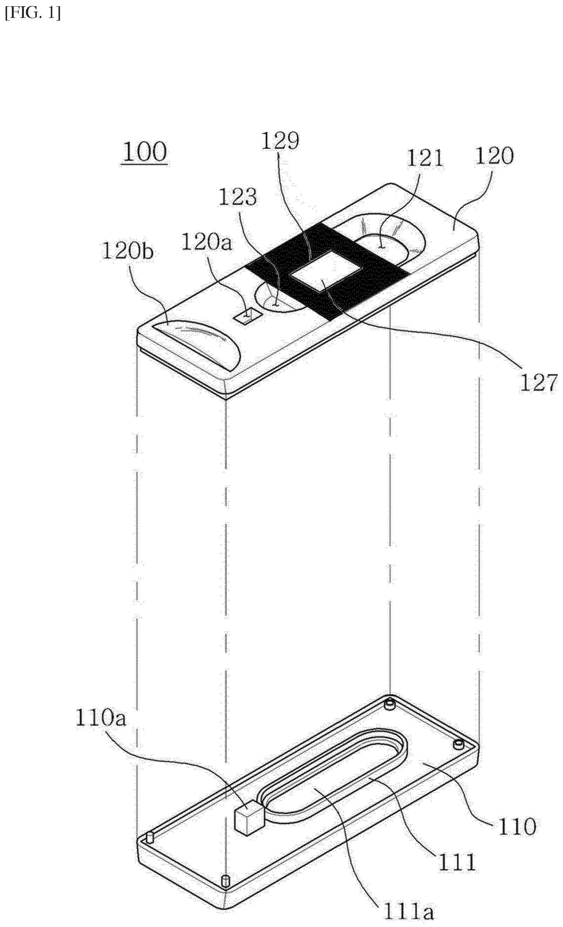

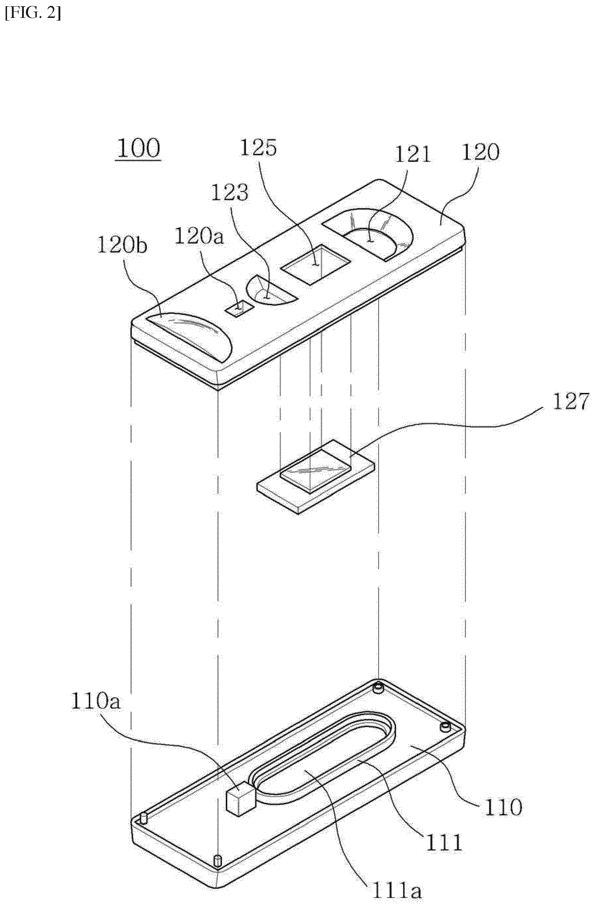

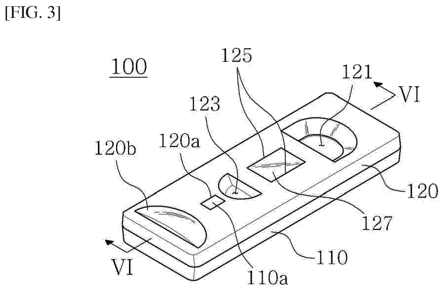

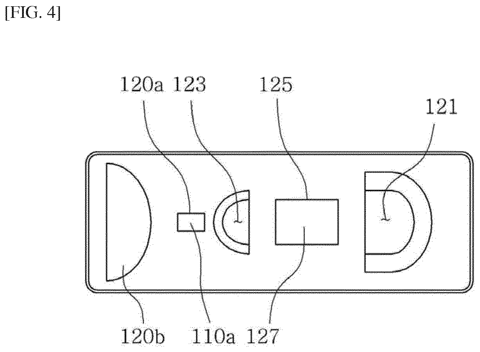

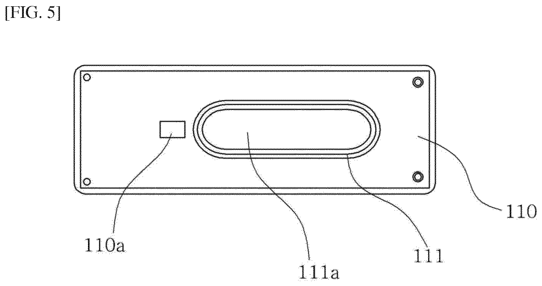

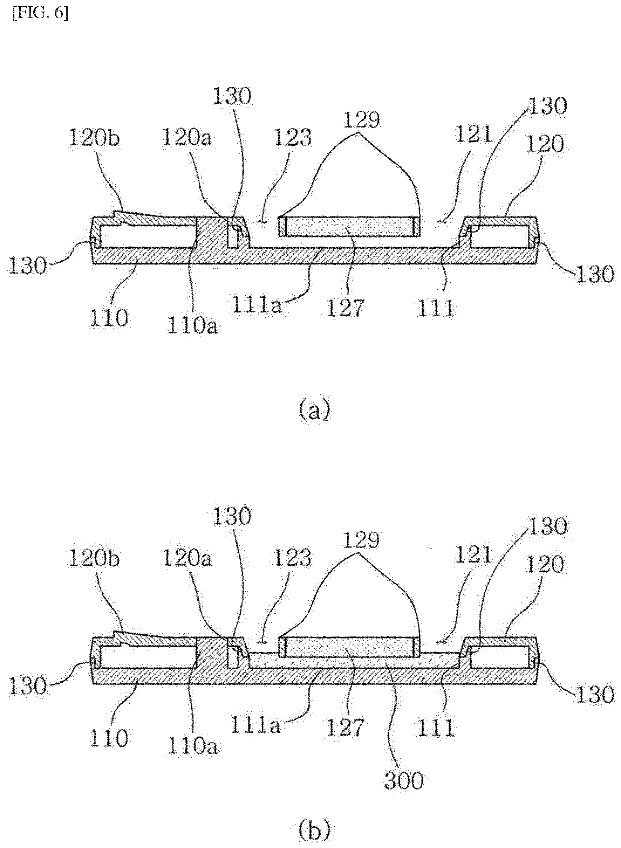

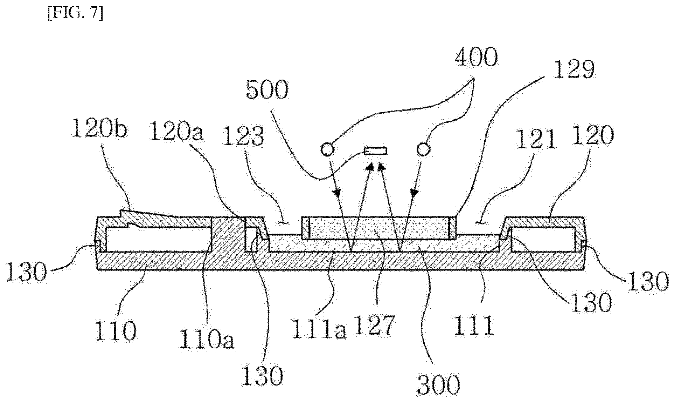

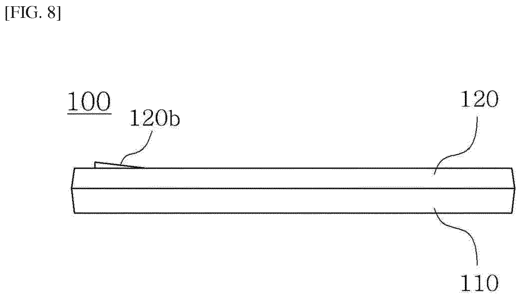

FIG. 1 is an exploded perspective view of the device for measuring reflective absorbance according to one embodiment of the present disclosure in which the light transmitting member are integrated with the cover member. FIG. 2 is an exploded perspective view of the device for measuring reflective absorbance according to one embodiment of the present disclosure in which the cover member comprises a window to which the transmitting member is engaged. FIG. 3 is a perspective view of the device for measuring reflective absorbance according to one embodiment of the present disclosure. FIG. 4 is a top view of the cover member of the device for measuring reflective absorbance according to one embodiment of the present disclosure. FIG. 5 is a top view of the base member of the device for measuring reflective absorbance according to one embodiment of the present disclosure. FIG. 6 is a sectional view taken along line VI-VI of FIG. 3 without (a) or with (b) a sample. FIG. 7 is a sectional view taken along line VI-VI of FIG. 3 schematically showing one exemplary light paths when using the device for measuring reflective absorbance according to one embodiment of the present disclosure. FIG. 8 is a side view of the device for measuring reflective absorbance according to one embodiment of the present disclosure.

As shown in FIGS. 1 to 8, the device for measuring reflective absorbance 100 according to one embodiment of the present disclosure comprises a base member 110 comprising a sample receiving part 111, and a cover member 120 covering a upper portion of the base member 110, and the cover member 120 comprises a sample inlet through which a sample is introduced into the sample receiving part and a light transmitting member 127, the light transmitting member being positioned perpendicular to the sample receiving part when the cover member 120 engaged with the base member 110, wherein the sample receiving part is configured to reflect an incident light illuminated through the light transmitting member.

The light transmitting member 127 of the present disclosure refers to a component through which a light is illuminated from a light source to a sample receiving part and also through which the light passed through a sample and reflected from the bottom of the sample receiving part is emitted. Referring to FIG. 1, the cover member and the light transmitting member may be integrally formed, in which case the cover member is formed of a light transmitting material, and the remaining area of the cover member, partially or entirely, may be configured to be light blocked. As depicted in FIG. 1, a light blocking member 129 may be configured to be present around the light transmitting member 127 when it is used to partially block the cover member from the light transmission, or may be used to block the entire area of the cover member except the light transmitting member. Or the cover member itself may be made of a material which is able to block the light from being transmitted. Herein light blocking or configured to be light blocked means that the cover member itself may be made of a light blocking material or the cover member may be made light impermeable by certain means, for example by physical means and/or an appropriate software. For example in the former case, the region of the cover member where the light blocking is desired is painted or printed black, or may be made opaque by use of a black film. In the latter case, for example, a reader that is used with the present device to read the analysis result is equipped with appropriate software that is able to selectively collect the light that has only passed through the light transmitting member.

Referring to FIG. 2, the light transmitting member 127 according to the present disclosure may be configured to be inserted into a window on the cover member and is positioned between the base member 110 and the cover member 120. In this case, all or part of the cover member may be fabricated to be light impermeable by being painted, printed, or filmed with black color after it being manufactured. For the insertion of the light transmitting member, a window 125 to which a light transmitting member is engaged is formed on the cover member 120 in a region which is perpendicular to the sample receiving part 111 when the cover member and the base member are engaged. When the light transmitting member 127 is engaged with the window, the cover member may include the light blocking member 129 by which all or part of the cover member is made light-blocked. Or the cover member may be made of a material which is able to block the light transmission.

In the present device, at least one, particularly more than one sample inlets 121 and 123 are provided. With one sample inlet, air may be introduced into and trapped in the sample receiving part 111 which prevents a sample being flowed into the sample receiving part or which can cause an inaccurate absorbance measurement. Thus, it is preferable to have at least two sample inlets or one sample inlet and one opening for air discharge. Therefore in one embodiment, the present device comprises a plurality of the sample inlets. For example, the device comprises two sample inlets 121 and 123 and the window 125 to engage the light transmitting part is interposed between the two sample inlets. In one embodiment, the device comprises two sample inlets positioned facing each other with the window 125 positioned therebetween.

Referring to FIGS. 1, 2, and 6, the light transmitting member 127 or the window 125 to which the light transmitting member is inserted is formed on a region of the cover member 120 which is located perpendicular to the sample receiving part 111 when the cover member covers the base member 110. Thus when the light transmitting member 127 or the window 125 to which the light transmitting member is engaged is formed between two sample inlets 121 and 123, the sample receiving part 111 also becomes located between the two sample inlets 121 and 123. Each of the sample inlets 121, 123 contained in the device may be of identical or different size. When the sample inlets comprised in the present device are of different size, one of the sample inlet which is larger in size than the others may be used for loading the sample, and the other inlet which is smaller in size may be used to discharge the air which has trapped/introduced while loading the sample into the sample receiving part 111.

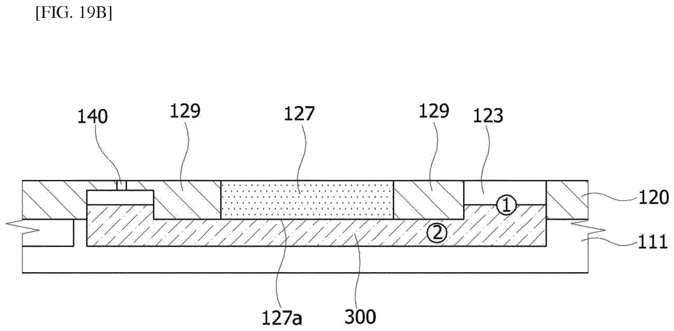

In this perspective, the present device further comprises an opening 140 in addition to the sample inlet. Referring to FIG. 19b, in one embodiment of the present disclosure, the device comprises one sample inlet and one opening 140 and they are positioned to face each other with the light transmitting member being positioned therebetween, and it is apparent to the skilled in the related art that their positions relative to each other may be changed. The opening according to the present disclosure can prevent the sample from being filled from the bottom of the sample receiving part covering the entire region of the bottom. That is, by the present device, the sample gradually fills the sample receiving part from one side thereof near the sample inlet, which makes the pressure generated inside pushed and discharged from the opening 140. This generates a sample flow from the sample inlet to the opening resulting in the removal of the air which may be generated and/or trapped in the sample receiving part during the sample loading. This resolves the problem caused by the air which results in the inaccurate absorption measurement. Openings which may be adopted for the present device are not limited to a particular size and a shape but rather may have various sizes and shapes as long as executing such functions as described herein. In one embodiment, the opening 140 of the present disclosure may have a diameter of about 0.1 mm to 2 mm.

Referring to FIGS. 6 and 7, when the cover member 120 is engaged with the base member 110, the cover member 120 and the base member 110 interlock with each other along the rim 130 thereof so that the device becomes substantially waterproof or sealed aerosol proof.

In the present disclosure, the sample 300 refers to a material, which is liquid, semiliquid or fluid substance, comprising a target(s) to be analyzed. It includes for example, various tissues/cells, bodily fluids, whole blood, serum, plasma, saliva, urine, sweat, hairs, and extracts derived therefrom. Further it includes for example, environmental samples e.g., air, soil, and water. In one embodiment, the samples which may be analyzed using the present device include blood, urine and saliva, and the blood includes whole blood, plasma, serum, or the blood that has been pretreated for a certain purpose for example to prevent its coagulation. Extracts from tissues and cells include for example carbohydrates, lipids, nucleic acids, and proteins or materials containing the same. The targets to be analyzed include, but are not limited to, markers related to a particular disease or conditions or states, for example, hemoglobin (Hb), C-Reactive Protein (CRP), Prostate Specific Antigen (PSA), Alpha-Feto Protein (AFP), Cancer Antigen 125 (CA-125), CA19-9, microalbumin, Hemoglobin A1c (Hb A1c), Cardiac Troponin I and T (cTn-I/T), total cholesterol, creatinine, glucose, uric acid, GPT(Glutamic Pyruvic Transaminase), and GOP(Glutamic Oxaloacetic Transaminase).

As shown in FIGS. 1, 2, 5, 6, and 7, the sample receiving part 111 may further include a reflective bottom portion 111a which reflects the light illuminated through the light transmitting member 127 from a light source.

The reflective bottom portion 111a of the present disclosure may be provided as separate component to be engaged with sample receiving part of the base member or the base member or the sample receiving part may be made of a material having a light reflective property. The reflective bottom portion of the present disclosure reflects the light illuminated through the light transmitting member, and the reflection includes a regular reflection or a scattered reflection. In one embodiment, the reflection is a scattered reflection. The reflective bottom portion 111a of the present disclosure or the base member or the sample receiving part having the above-described reflection property may be formed of a material which reflects the light illuminated through the light transmitting member 127, particularly formed of a material that is able to generate a scattered reflection. The material may be appropriately selected in consideration of the types of light source 400 used. For example, when an LED or a laser beam is used as the light source, the reflective material may be any white polymer material, which may include, for example, Acrylonitrile Butadiene Styrene (ABS), Polystyrene (PS), Polyethylene (PE), and Polypropylene (PP). Alternatively, metals such as aluminum, titanium, or chromium with a smooth reflection surface having a regular reflection characteristic may also be used. Above examples are just exemplary and the reflective bottom portion 111a is not limited thereto.

For example, referring to FIG. 7, the absorption is measured using not a transmitted light but a reflected light, thus the light illuminated is reflected from the reflective bottom part 111a and travels back through the light transmitting member 127 which is then collected by a light detector.

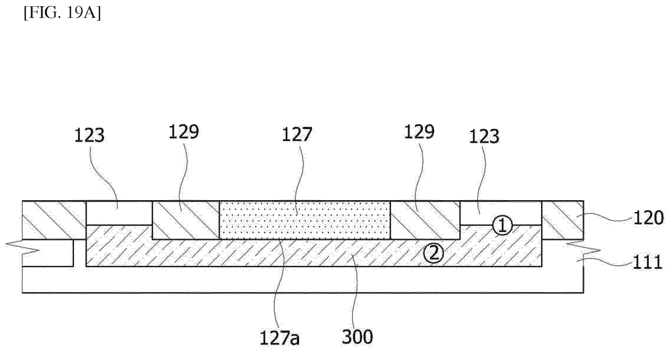

Referring to FIGS. 6 and 19A, the sample 300 introduced into the sample receiving part 111 through the sample inlets 121 or 123 resides in the sample receiving part 111 as shown in FIG. 6 (b), where the surface of the sample is at least to be contacted with or immerses the bottom of the light transmitting member 127 so that no air layer is present underneath the bottom of the light transmitting member 127b. Referring to FIG. 7, this is advantageous for obtaining an accurate result because the absorption is measured on the light which has passed and reflected through a sample with a uniform depth. In one embodiment, the present device is configured in such a way that the sample is introduced through the sample inlet 121 or 123 to fully fill the sample receiving part 111 and at least to be contacted with the bottom of the light transmitting member 127.

Also as long as the sample is introduced in such a way that its surface at least touches the bottom of the light transmitting member, it is not necessary to precisely measure the volume of the sample 300 to be loaded upto one microliter unit and instead the sample 300 is taken in an amount enough to fill at least upto the bottom of the light transmitting member 127. This is advantageous because it can negate the error due to a variation in the minute amount of sample loaded and thus increase the user convenience and provide accurate and consistent results. For example, as long as a user loads the certain amount of sample 300 enough to fill the sample receiving part 111 at least to touch the bottom surface of the light transmitting member, the error due to the variation in the amount of sample volume is minimized. The particular loading volume to meet such condition may vary depending on the specific dimensions of the device employed. For example, when the present device is made to be used with the reader *i-Chroma* (Boditech Med Inc., ChunCheon, Korea), the present device may have the dimensions of 1.5 cm (width) x 4 cm (length) x 0.4 cm (height), in which case the loading volume of the sample may be in the range of about 50 microliters to about 250 microliters, in particular, about 100 micrometers.

In this case, the size of the device refers to a size of the reflective absorbance measuring device 100, and the size of the integral device 200 for measuring reflective absorbance and lateral flow assay, which is described hereinafter, may have the dimensions of about 1.5 cm (width) x 9.3 cm (length) x 0.4 cm (height), but without being limited thereto.

The size of the light transmitting member 127 and the window 125 to which it is engaged may vary depending on the size of the device, the types and sizes of the light source 400 and the light detector 500 employed, and the types of material with which the light transmitting member 127 is made of. In one embodiment, when the present device is made to be used with the reader *i-Chroma* (Boditech Med Inc.), the size of the window 125 may be about 0.5 cm (width) by 0.7 cm (length).

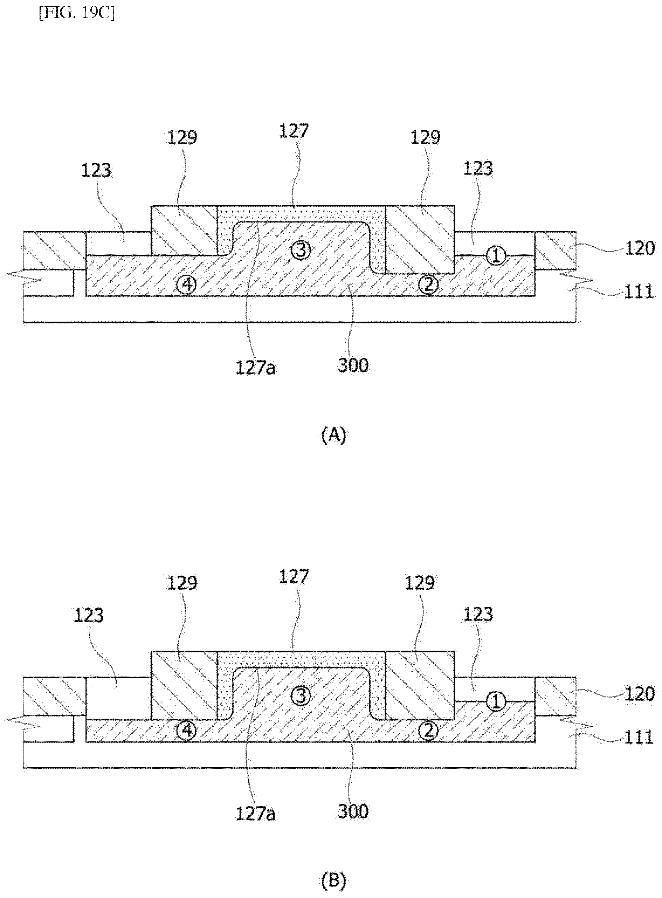

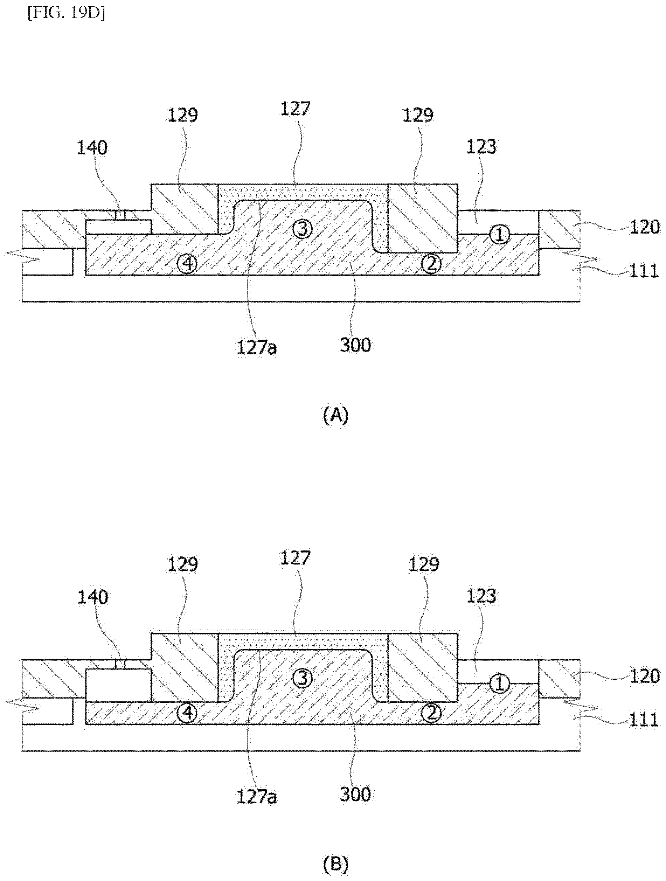

In other aspect of the present disclosure, the sample may be loaded in such a way that the surface of the sample 300 may not reach/touch the bottom 127a of the light transmitting member 127 depending on the particular structures of the bottom of the light transmitting member 127 takes and types of the sample 300 analyzed. The bottom 127b of the light transmitting member may be flat as in FIGS. 19A and 19B, or be recessed inward as in FIGS. 19C and 19D. In the latter case, the distance (③) between the sample receiving part and the bottom surface of the light transmitting member increases and the bottom surface 127b of the light transmitting member becomes higher in position than the surface of the sample (①), which is particularly useful for the accurate measurement of the concentration of some samples depending on the types of sample analyzed.

The particular distance between the bottom surface of the light transmitting member and the sample receiving part may vary depending on the types of sample used, the type and/or amount of the targets to be detected, and/or the sensitivity and/or specificity of the detection, or it also may vary depending on the specific dimensions of the devices used. In one embodiment, the distance may be in the range of about 0.5 mm to 10 mm. In other embodiment, the distance is about from 0.5 mm to 3.0 mm. In another embodiment, the distance is about from 1 mm to 2 mm.

Referring to FIGS. 19C and 19D, the distance ② and ④ under each side of the light transmitting member 127 may be different or identical. In one embodiment of the present disclosure, the distance underneath the left and right sides of the light transmitting member 127 are different, and in this case, the sample fills the sample receiving part in such a way that air is not generated underneath the bottom of the light transmitting member. This is due to an capillary action generated by the space ② and/or to the pressure generated from the sample loading. By the movement of the sample loaded which gets initiated by the capillary action generated by the space ②, the sample gradually fills the sample receiving part following the surface defined by the space ③ from the sample inlet as the pressure builds up with the sample loading, which results in the reduction in the air generated or trapped.

The light transmitting member having the bottom surface which is not flat as described above also may be employed with one or more sample inlets for example, inlets 121 and 123, and/or opening 140. Referring to FIGS. 19C and 19D, the light transmitting member 127 is interposed between each of the two sample inlets or between the sample inlet and the opening. In the latter case, the sample gradually fills the sample receiving part starting from the sample inlet side while the pressure generated is discharged through the opening, and thus any air which may be present in the sample receiving part are removed.

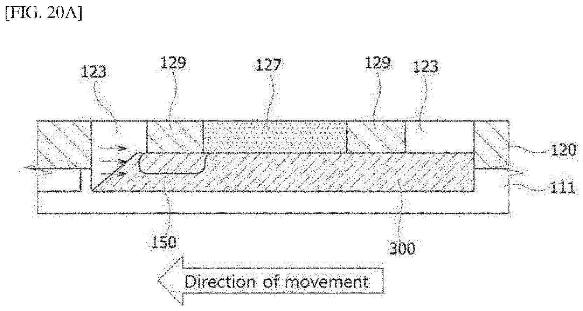

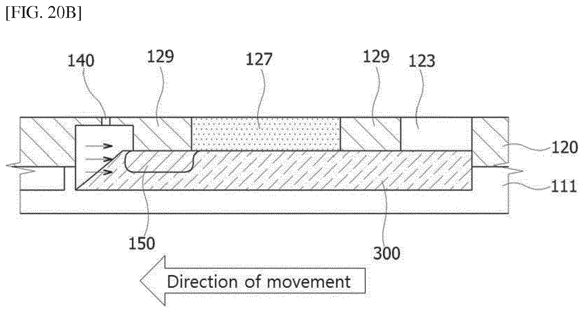

In another aspect of the present disclosure, the absorption measuring device of the present disclosure may further comprises a protrusion 150, which is useful to prevent the air generated underneath the bottom of the light transmitting member within the sample receiving member as described below. That is, the air may be generated when the device is under the influence of some external force such as during the transfer/movement of the device, in which case the force exerted in a direction that is opposite to the movement makes the sample in the sample receiving member moves to one side in a direction as depicted as arrows in FIGS. 20A and 20B. This results in the formation or trapping of the air underneath the bottom of the light transmitting member. The protrusion 150 can prevent such air formation by generating a capillary force in a direction that is opposite to that of the sample flow generated by the external force, which results in the prevention of unwanted sample flow. Referring to FIGS. 20A and 20B, the protrusion 150 may be formed on the bottom surface of the cover member adjacent to the bottom of the light transmitting member while not preventing the light path. Further, various structures may be adopted for the protrusion 150 as long as it exerts the function as described above.

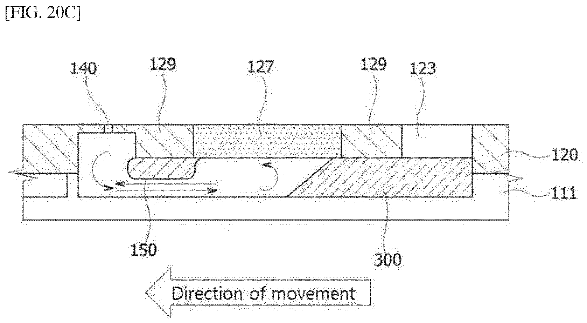

As shown in FIGS. 20A to 20C, when the present device is provided with the protrusion 150, one or more sample inlets 121 and 123 and/or openings 140 may also be used, in which case the light transmitting member 127 is interposed between each of the two sample inlets or between the sample inlet and the opening. In the latter case, the sample gradually fills the sample receiving part starting from the sample inlet while the pressure generated is discharged through the opening, and thus any air which may be present, introduced or trapped in the sample receiving part gets removed. Further the protrusion 150 also prevents the air formation as described above (Refer to FIG. 20C).

In another aspect, the present disclosure provides a system for measuring reflective absorbance, the system comprising: the device 100 as described above, a light source 400 being disposed above the cover member 120 of the device; and a light detector 500 being disposed above the cover member, the detector measuring the amount of light being reflected from the sample receiving part to which a light is illuminated through the light transmitting member from the light source. The system of the present disclosure is advantageous because it measures the reflected light and thus the components of the system may be arranged to locate at one side of the system which results in a system which is convenient to manufacture and smaller in size compared to the system which detects a transmitted light (Refer to FIG.7)

In that case, one or more light sources 400 may be provided if necessary. The light source which may be used for the present disclosure may include, but is not limited to, for example, Light Emitting Diode (LED), Ultraviolet (UV) LED and laser. For example, when hemoglobin is measured, an LED having a wavelength of 540 nm may be used. However, various wavelengths may be used depending on the types of targets analyzed. For example, a UV light having a wavelength of 255 nm to 380 nm may be used to measure nucleic acids or proteins in the sample. The light detected by the light detector 500 is converted to a concentration of the target being analyzed by using methods known in the art.

Further, it is preferred that the light source 400 and the light detector 500 are not longitudinally positioned in a row, instead the light sources 400 are disposed askew on each side of the light source.

Accurate detection is possible when the surface reflected light, that is, the light reflected from an upper surface or a lower surface of the light transmitting member 127 is minimized. Therefore, as shown in FIG. 7, in one embodiment, the light sources 400 and light detector 500 is horizontally positioned.

The light transmitting member 127 is configured to be formed extending in one direction from the cover member 120, and the sample receiving part 111 is also configured to be formed extending in one direction from the base member 110.

That is, to arrange the light source 400 and light detector 500 in a way to minimize the influence from the surface reflected light, the window 125 and the sample receiving part 111 may configured to be formed/positioned extending in one direction. Then the light source 400 and light detector 500 may also be positioned along that direction in a way to minimize the adverse effect from the surface reflected light. In that case, the light source 400 and the light detector 500 are not longitudinally positioned in a row as described above. When the light transmitting member 127, the window 125, and the sample receiving part 111 are configured to be positioned in one direction, the device is able to be used with systems equipped with a light source in various positions.

The reflective absorbance measuring device 100 according to the present disclosure may be manufactured to have various forms and sizes depending on the type of the reader used. For example, the reflective absorbance measuring device may have a bar or rectangular shape, and may be used in a variety of devices measuring absorption (readers) and the present device may be manufactured to have various sizes accordingly. The reflective absorbance measuring device 100 according to the present disclosure is compatible to be used with a variety of readers for measuring absorption in a reflective manner, which may include, without limitation, for example, *i-Chroma* (Boditech Med Inc.) and coagulometer and the like.

As shown in FIGS. 1 to 8, the absorption measuring device according to the present disclosure may further include a tetragonal hole 120a, and may also be manufactured not to have a tetragonal hole. The tetragonal shape 110a and the tetragonal hole may be used as a standard reflective surface to generate a signal for background calibration. Further the tetragonal hole 120a and the tetragonal shape 110a are also used to engage the base member 110 and the cover member 120.

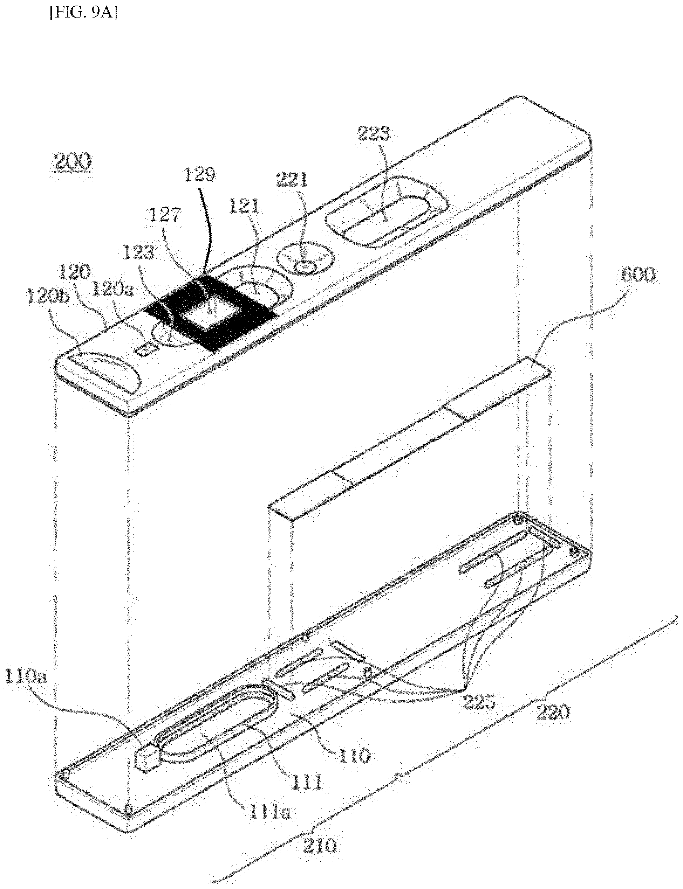

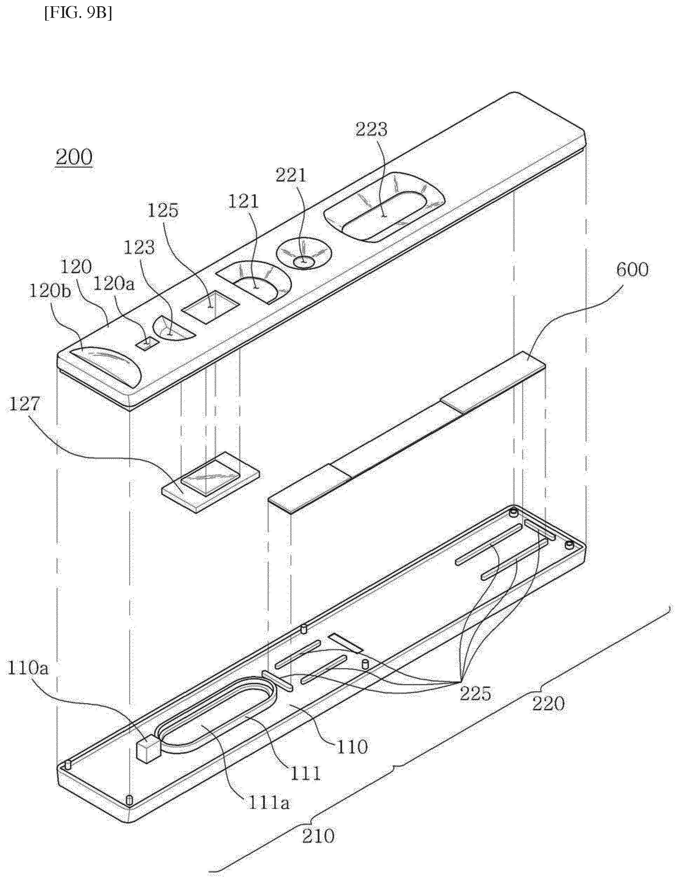

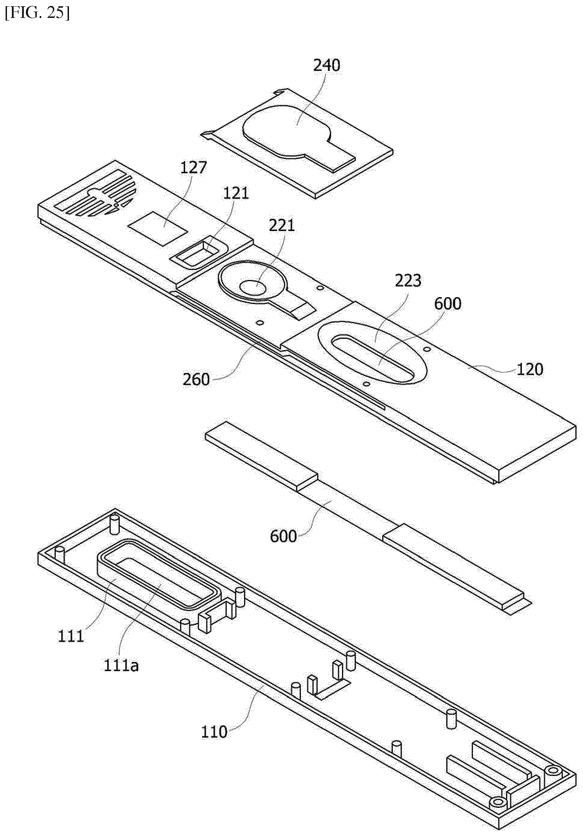

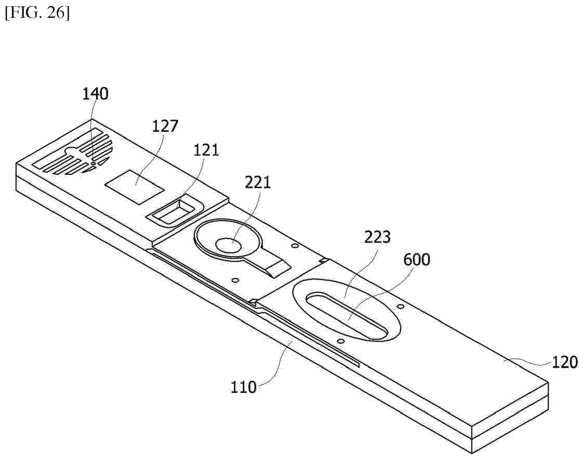

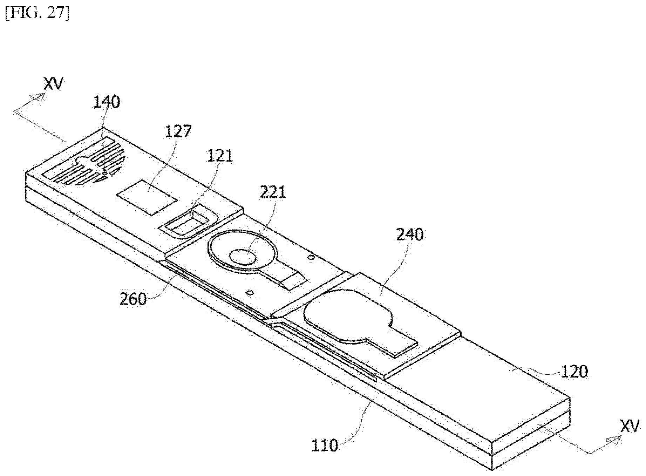

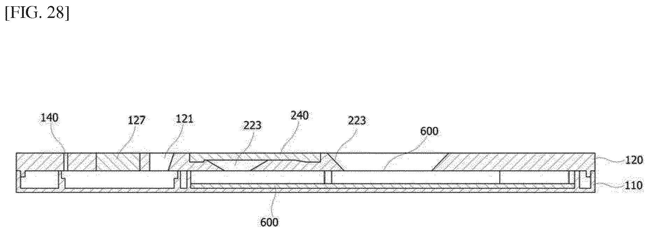

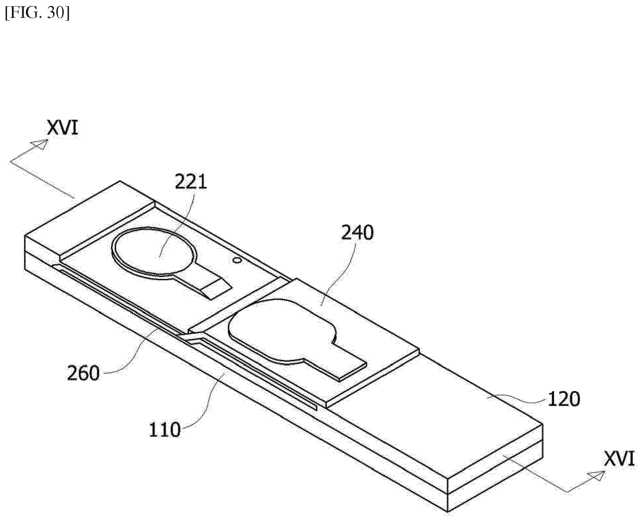

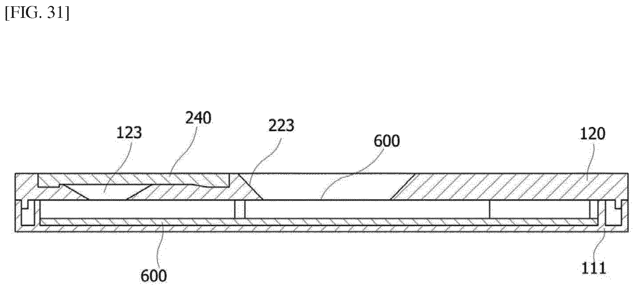

The semicircular protrusion 120b may be used for convenient holding of the device when handling the device, which is also true with the tetragonal hole 120a, the tetragonal shape 110a as shown in FIGS. 9 to 16, 22, and 25 to 31.

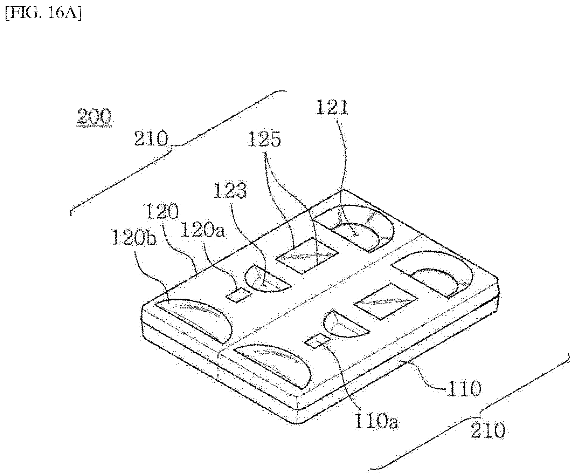

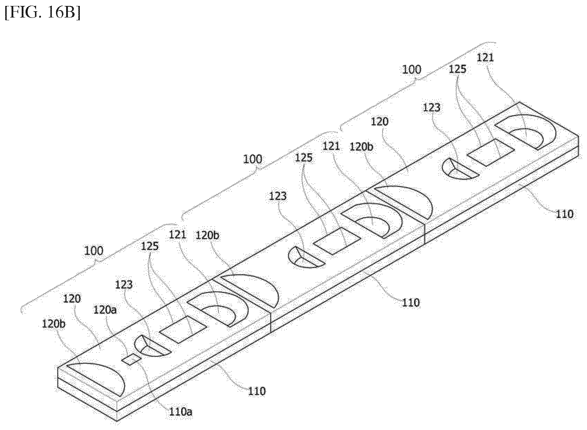

In another aspect, the present disclosure also relates to an integrated device for measuring reflective absorbance where multiple devices are physically joined in various combinations in terms of their relative positions. Referring to FIGS. 16A and 16B, in one embodiment, the integrated device comprises two devices and each device is arranged side by side. In other embodiment the integrated devices comprises three devices and each device is arranged in a row.

In another aspect, the present disclosure provides a device for lateral flow assay which comprises a base member comprising a strip receiving part; a cover member covering an upper portion of the base member and comprising a sample inlet and a window for measurement; and a window cover for opening and closing the window, wherein a sample is introduced into a strip mounted on the strip receiving part through the sample inlet and the window cover is positioned being perpendicular to the strip receiving part when the window is closed.

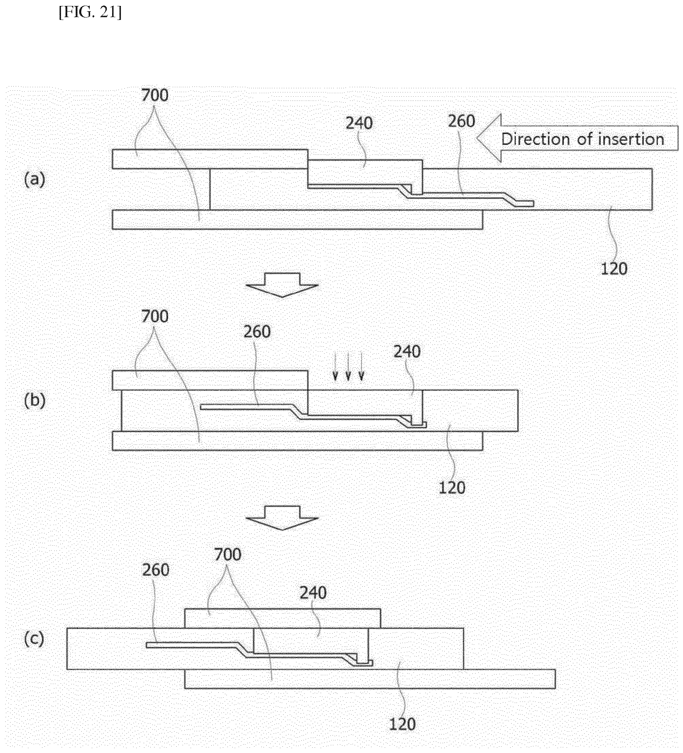

In the present disclosure, the window cover is a detachable or removable, and the window cover may be opened or closed in a sliding manner in which the window cover is pushed forwards and backwards or the window cover may be opened or closed by moving the window cover upward and downward. In one embodiment, the window cover is opened by sliding, and referring to FIG. 21, the window cover is automatically pushed open when the device is inserted into a reader for reading. It is understood that the window cover may employ various shapes as long as it exerts its function of opening and closing the window. For other uses and structures of the window cover, it may be referred as described hereinafter.

In another aspect, the present disclosure relates to an integrated device for measuring reflective absorbance and lateral flow assay. Here, the same reference number refers to the same element throughout the drawings in the various views and the drawings to depict various aspects of the present disclosure and the detailed explanation for the elements described hereinbefore will be omitted to avoid repetition. Further, when the reflective absorbance measuring device 100 is integrated in a device 200 for measuring reflective absorbance and lateral flow assay, the reference number 210 is used instead of 100 starting from FIG. 9.

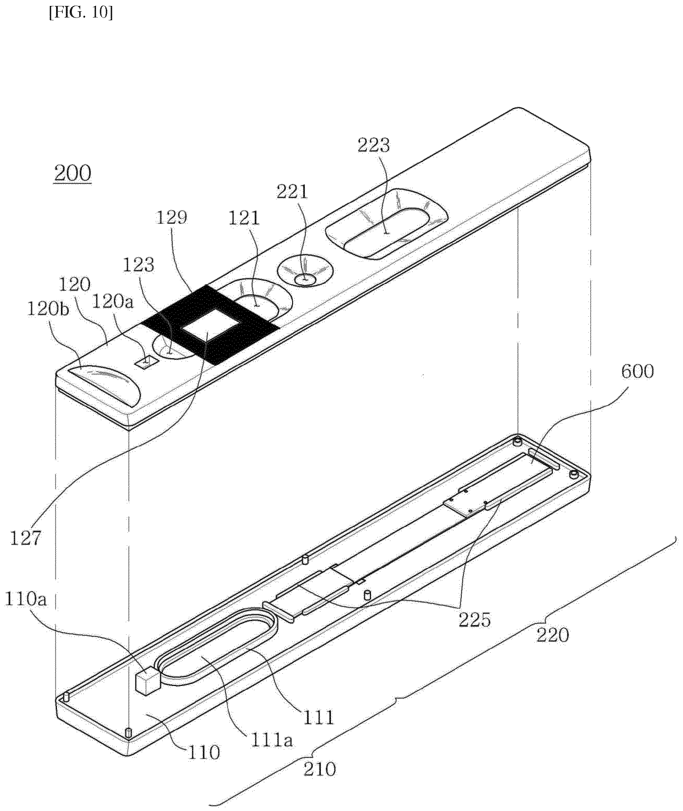

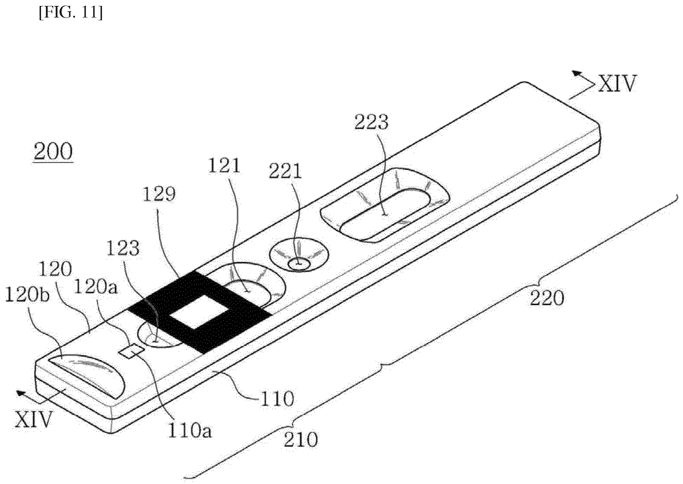

FIGS. 9A and 9B are an exploded perspective view of an integrated device for measuring reflective absorbance and lateral flow assay, wherein the light transmitting member is formed being integrated with the cover member (9A) or the cover member comprises a window to which the light transmitting member is engaged (9B). FIG. 10 is an exploded perspective view showing the integrated device for measuring reflective absorbance and performing lateral flow assay according to one embodiment of the present disclosure with a strip mounted. FIG. 11 is a perspective view of the integrated device for measuring reflective absorbance and performing lateral flow assay according to one embodiment of the present disclosure in which the cover member and the base member are engaged to each other closing the device.

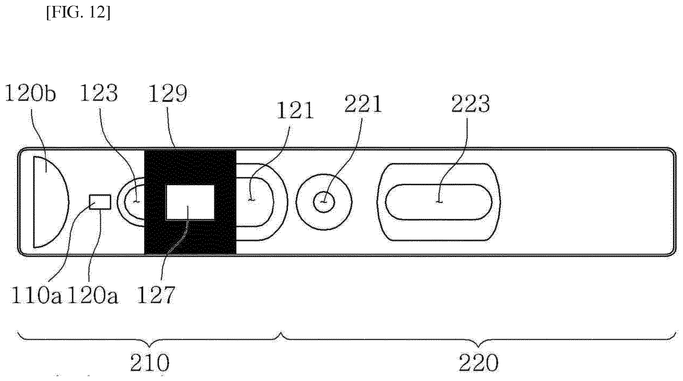

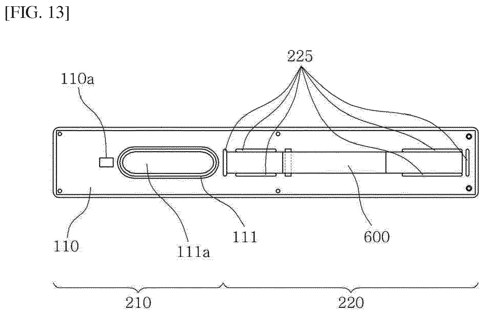

FIG. 12 is a top view of the cover member of the integrated device for measuring reflective absorbance and performing lateral flow assay according to one embodiment of the present disclosure. FIG. 13 is a top view of the base member of the integrated device for measuring reflective absorbance and performing lateral flow assay according to one embodiment of the present disclosure.

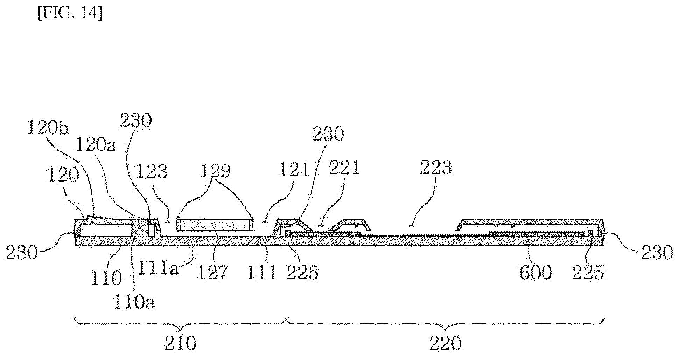

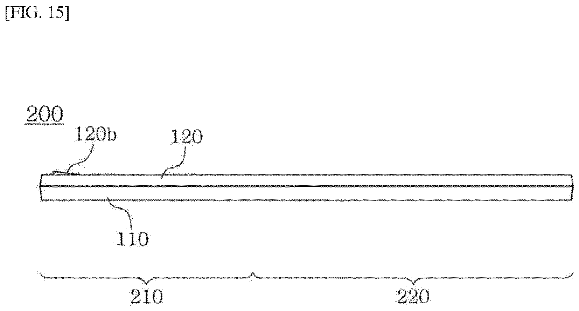

FIG. 14 is a sectional view taken along line XIV-XIV of FIG. 11. FIG. 15 is a side view of the integrated device for measuring reflective absorbance and performing lateral flow assay according to one embodiment of the present disclosure.

As shown in FIGS. 9 to 15 and 22 to 28, the integrated device 200 for measuring reflective absorbance and lateral flow assay according to another embodiment of the present disclosure is used both for measuring reflective absorbance of the sample 300 and also for performing lateral flow assay in which a target analyte in the sample 300 is quantitatively and/or qualitatively measured. In the integrated device 200, the device for measuring reflective absorbance 210 and the device for performing lateral flow assay 220 are physically joined to each other, and they are arranged to be adjacent to each other in a variety ways depending on the structures and shapes of a reader used. For example, in one embodiment of the present disclosure, the reflective absorbance measuring device 210 and the lateral flow assay device 220 may be arranged in a row in lengthwise as shown in FIGS. 9 to 15, or they may be arranged side by side (Not shown).

In the present disclosure, the term in a row means the devices are arranged longitudinally, and the term side by side means the devices are arranged horizontally.

Further, the integrated devices may comprise one or more reflective absorbance measuring devices 210 and one or more lateral flow assay devices 220. For example, the integrated device comprising two reflective absorbance measuring devices 210 and one lateral flow analyzing device 220 may be provided. When they are arranged in a row, two reflective absorbance measuring devices 210 are positioned in a way flanking one lateral flow assay device 220 in the middle. Alternatively, two reflective absorbance measuring devices 210 are arranged in a row continuously extending from top to bottom followed by one device 220 (or one device 220 followed by two devices 210 in a row). Or when they are arranged side by side in parallel, each of the two reflective absorbance measuring devices 210 is positioned left and right respectively with one lateral flow assay device 220 in the middle. Alternatively, two reflective absorbance measuring devices 210 are arranged on the left side(or on the right side) and one device 220 is arranged on the right side (or on the left side). In addition, the devices 210 and 220 in the integrated device may take a variety of positions relative to each other depending on the structure/shape of the reader that is used.

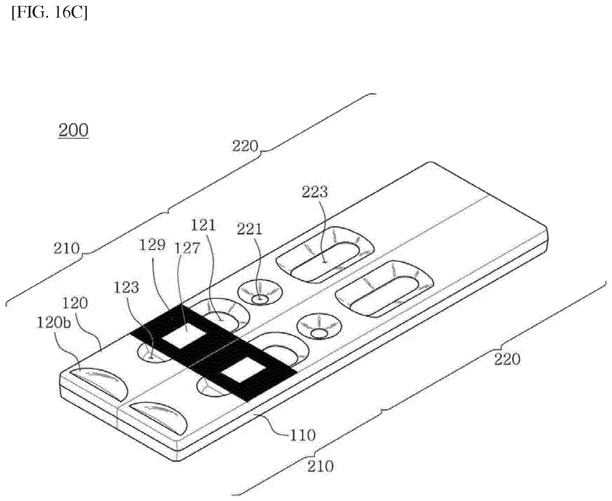

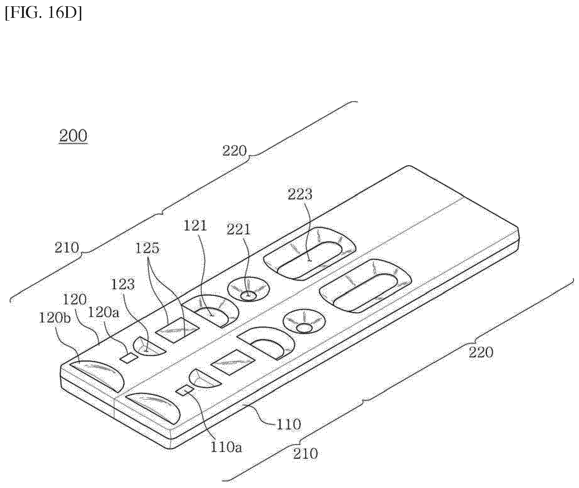

FIGS. 16C and 16D are perspective views of the integrated devices for measuring reflective absorbance and analyzing lateral flow assay according to another embodiment of the present disclosure, and the former shows a device wherein the light transmitting member is inserted into the window and the latter shows a device wherein the light transmitting member is configured to be integrally formed with the cover member.

Further, the integrated device may comprise two or more reflective absorbance measuring devices 210 and two or more lateral flow analyzing devices 220. For example, as shown in FIGS. 16B and 16C, two reflective absorbance measuring devices 210 and two lateral flow analyzing devices 220 are provided, in which one reflective absorbance measuring device 210 and one lateral flow analyzing device 220 are arranged in a row and the other reflective absorbance measuring device 210 and lateral flow analyzing device 220 are arranged in a row, which in turn are arranged side by side. In other words, the reflective absorbance measuring devices 210 disposed side by side and the lateral flow analyzing devices 220 disposed side by side are in turn disposed in a row.

Thus, the integrated device of the present disclosure in which the reflective absorbance measuring device 210 and the lateral flow analyzing device 220 are integrated into one device, provides rapid and convenient analysis of the sample by measuring absorbance and lateral flow assay at once.

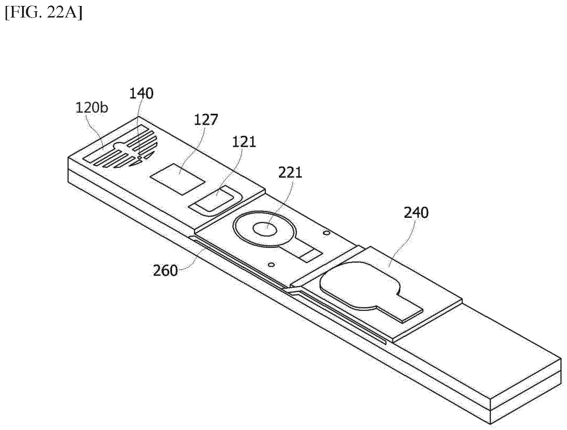

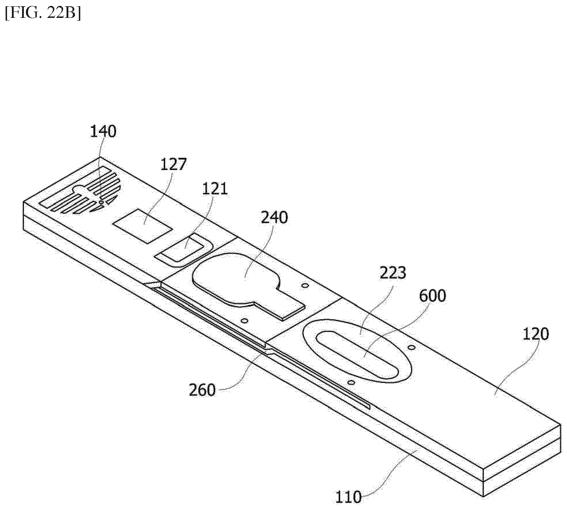

As shown in FIGS. 9 to 16, 22, and 25 to 28, the integrated device according to the present disclosure includes a device for lateral flow assay which comprises a base member and a cover member extending from the device for measuring reflective absorbance wherein the cover member formed on the lateral flow assay device comprise a second sample inlet and a window, the base member formed on the lateral flow assay device comprises a strip receiving part, and a sample is introduced into a strip through the second sample inlet, the strip being mounted on the strip receiving part. In one embodiment of the present disclosure, as shown in FIGS. 22 to 27, the second sample inlet may be formed adjacent to the sample inlet formed on the cover member of the reflective absorbance measuring device.

Meanwhile, the lateral flow assay is a method for quantitatively or qualitatively measuring an analyte contained in the sample 300, and in which, for example, a sample is applied to a cellulose nitrate membrane to which an antibody and/or an antigen that is specifically binding to the analyte of interest are coupled at a certain location and then the sample applied is moved by chromatographic flow during which a protein or analyte of interest is captured by the antigen-antibody binding forming a complex which is then detected.

Referring to FIG. 11, if the base member 110 formed throughout the reflective absorbance measuring device 210 and the lateral flow analyzing device 220 is covered by the cover member 120, they interlock with each other along the rim 230 thereof and the device becomes substantially waterproof or sealed aerosol proof.

Referring to FIGS. 9 to 15, 22, and 25 to 28, basically, the lateral flow analyzing device 220 is disposed adjacent to the reflective absorbance measuring device 210, and includes a separate second sample inlet 221 and a window 223 formed on the cover member 120, and includes a strip receiving part 225 formed on the base member 110 to accommodate the strip 600. The window 223 is a structure for measuring/identifying a reaction result, for example, an antigen-antibody reaction from the lateral flow analysis on the strip.

In one embodiment of the present disclosure, the lateral flow analyzing device 220 may further comprise a window cover 240.

The window cover according to the present disclosure protects the strip mounted on the strip receiving part. The strip which may be used for the present disclosure is known in the art and includes for example, a cellulose nitrate membrane, but is not limited thereto. For example, the window cover is able to prevent the strip from being damaged, contaminated or wetted due to the temperature changes particularly during transportation.

Referring to FIGS. 22A and 22B, the window cover 240 is coupled to the cover member 120 to open or close the window 223 such that it closes the window when the device is not in use or before it is being inserted into a reader, and it exposes the window by being slid semi-automatically when it gets inserted into a reader. Or the window cover 240 may be configured to open or close the window manually.

Referring to FIGS. 21, 22A, and 22B, the window cover 240 according to the present disclosure may be opened or closed, for example, by sliding or by pulling it up and down. In the former, for example, the window cover 240 closes the window until the device is used for a lateral flow assay, then the cover is automatically opened when it is inserted into a reader for detecting the result of the lateral flow assay. In the latter case, for example, the cover is opened or removed manually before the device being inserted into a reader.

In one embodiment, the window cover is opened or closed by sliding, in which, as described above, the window cover may be opened manually or automatically by sliding when the lateral flow assay is done. In the latter case, the window cover is opened automatically while the device being inserted into a reader as shown in FIG. 21.

Referring to FIG. 21, the window cover 240 is positioned at a height higher than a distance of the inner space of the housing 700, so when the device is inserted into a reader, the window cover 240 gets pushed backward by the housing and at the same time moved backward along the cover moving guides 260 formed on each side of the cover member 120 so as to be positioned at the same height as the inner distance of the housing and to expose the strip mounted on the strip receiving part.

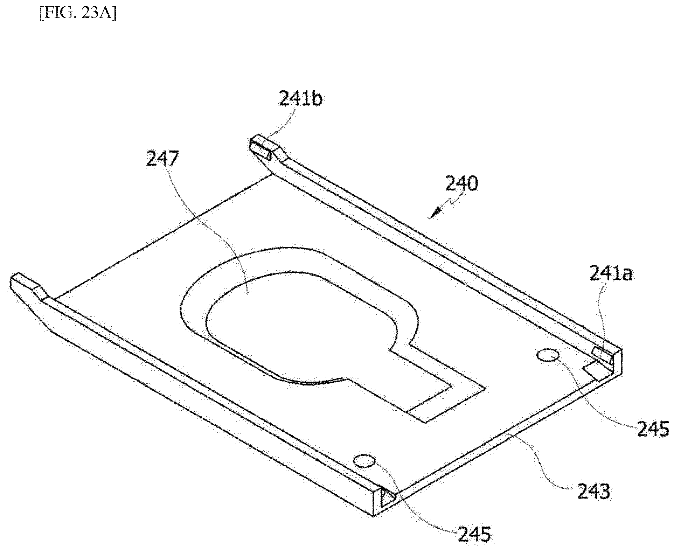

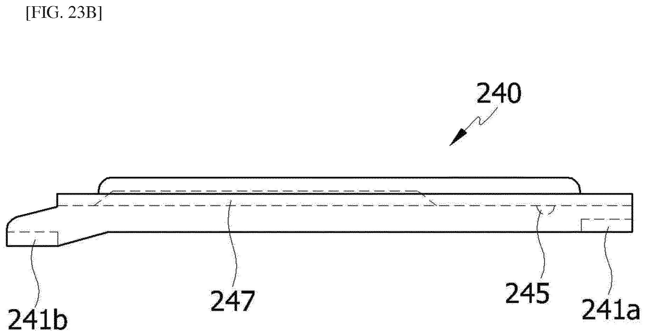

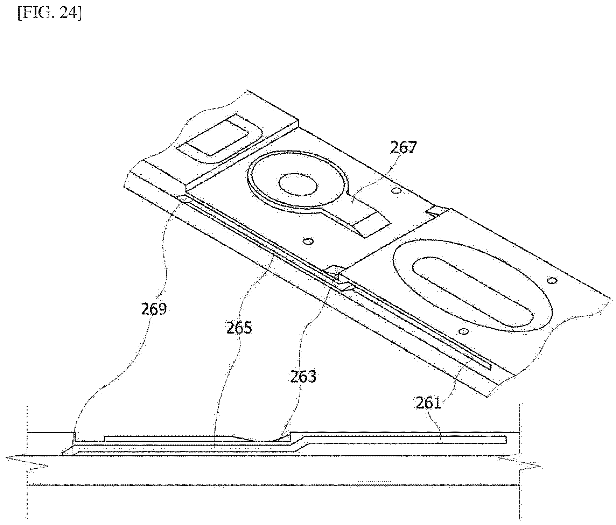

Referring to FIGS. 23 and 24, when the window cover 240 is configured to be operated in a sliding manner by semi-automatically, the measurement window cover 240 is provided with an appropriate structure for sliding, and the cover member is provided with a moving guide 260 on each side thereof.

Referring to FIG. 23, the measurement window cover 240 comprises a first and a second guide protrusion 241a and 241b, respectively, which are able to move along the moving guides 260. The second guide protrusion 241b is formed below the first guide protrusion 241a so as to be positioned downwards during the sliding thereof. A stop protrusion 245 is engaged with a recess (not shown) formed on the cover member 240 to prevent the window cover from being removed by accident when the window cover 240 closes the window under the minor impact. A trapping sill 243 comprises a sloped groove or a groove at a right angle at each end to prevent the window cover from being moving forward and to cover back the window and the strip receiving part after it has opened the window by sliding. The receiving recess 247 has a stepped structure for adjusting the amount of the sample introduced into the second sample inlet 221.

Referring to FIG. 24, the moving guide 260 for the window cover for semi-automatically opening or closing the cover in a sliding manner is formed on each side of the cover member. Specifically, a first guide groove 261 is a structure for docking the window cover 240, and a rail 265 for sliding is formed along the side. The second guide groove 263 is a structure for preventing the measurement window cover 240 from moving in a reverse direction once it has been pushed back and become in a position to expose the strip and been caught by the sloped recess formed on each end of the trapping sill 243 of the window cover 240. Also, a fourth guide groove 269 is provided to prevent the window cover from being removed from its position after it has been moved to open the window and o expose the strip. A third guide groove 267 is provided to prevent the window cover 240 from getting caught by the second sample inlet 221 and thus to provide smooth movement thereof.

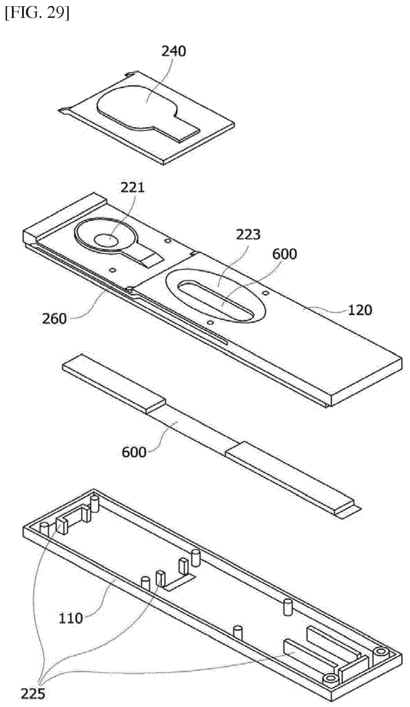

As shown in FIGS. 29 to 31, the lateral flow analyzing device included in the integrated device according to the present disclosure may be used by itself. The lateral flow analyzing device according to the present disclosure comprises a window cover that may be opened and closed as described above, and its characteristics, the way it works, and its uses are as described above.

The devices of FIGS. 1 to 16 and 19 to 31 according to the present disclosure are exemplary only and also the shapes of the elements/components employed therein also are exemplary, and it will be appreciated by the skilled person in the relevant art that various shapes are also encompassed by the present disclosure.

The absorption measuring device, the lateral flow analyzing device, and the integrated device including the same according to the present disclosure may be made of a variety of synthetic resins which are chemically stable or a combination thereof. For example, the present devices may be made by fabrication methods known in the art using a variety of thermoplastic or thermosetting plastics such as, without being limited thereto, polyethylene, polypropylene, polystyrene, polyethylene terephthalate, polyamide, polyester, polyvinyl chloride, polyurethane, polycarbonate, polyvinylidene chloride, polytetrafluoroethylene, and polyetherimide, and a combination thereof. Meanwhile, materials for fabricating the present device are not necessarily limited to a specific material or a specific group of material, but any material suitable for the purpose of the present device may also be used. The device according to the present disclosure may be manufactured using various molding methods known in the art, for example, injection, rotation, extrusion, and/or calendaring methods depending on the type of the material used. In one embodiment of the present disclosure, the cover member and the base member of the device may be made of Acrylonitrile Butadiene Styrene (ABS), and may be manufactured by injection-molding of acryl when a transparent material is used. Those skilled in the art would be able to select materials and methods appropriate for the purpose of the present disclosure from various materials and methods known in the art to manufacture the device according to the present disclosure. In addition to the synthetic resins, various additives for example, fillers, plasticizers, stabilizers, coloring agents, and antistatic agents may also be used as required.

The lateral flow assay according to the present disclosure uses in one embodiment immunochromatography. A variety of strips 600 (see FIG. 9) that are known in the art may be used for the lateral flow assay device 220 according to the present disclosure. For example, those disclosed in Korean Patent Application Publication Nos. 2009-0006999 and 2005-0072015 may be used without being limited thereto. The strips disclosed therein comprise a sample pad to which a sample is applied, a releasing pad comprising a detection antibody, a membrane for chromatography (mainly, nitrocellulose membrane) for sample movement/separation and antibody-antigen binding, and an absorption pad for keep the movement of the sample in one direction. The detection antibody is fixed to, for example, colloidal gold particles for detection labelling. Latex beads or carbon particles may also be used instead of the gold. A diagnosis kit based on the lateral flow assay is generally designed to detect an analyte in the form of a sandwich. The analyte in the liquid sample starts moving by the application onto the sample pad and reacts and forms a complex with a detection antibody, which is not fixed, in the releasing pad and then the complex moves through the membrane. The complex again reacts with a capture antibody fixed on the membrane, which results in the sandwich formation at the fixed site on the membrane. Proteins are visually transparent and thus the complex formation and the relative amount of the analytes in the sample are determined by the amount of gold particles to which the complex associated.

The present integrated device 200 may be manufactured in various shapes and sizes, and in one embodiment, the present device has a rectangular shape, and may be used with a variety of absorption measurement devices (readers) that is compatible. The reader which may be used with the present integrated device 200 includes, but is not limited to, *i*-Chroma (Boditech Med Inc.), Triage System (Biosite Inc., Sweden), and RAMP System (Response Biomedical Inc. Canada). The present device may be applied in cases where the analytes are analyzed both by absorption and lateral flow assay.

The reflective absorbance measuring device 210 may be used to detect and/or quantitatively measure various biological materials. For example, it includes, but is not limited to, the detection of hemoglobin (Hb), microorganisms, proteins, and DNAs, and other color changes by the addition of enzymes/catalysts, or detection of water pollution using BOD or COD, GPT/GOT for liver condition, enzyme activities using NADH generation (e.g., ADH alcohol dehydrogenase, antioxidant activity) and the like.

The lateral flow assay device 220 may be used for quantitative and/or qualitative detection of various biological materials including, for example, hsCRP(high sensitivity C-reactive protein), MicroCRP, HbA1c (Glycated hemoglobin), microalbumin, PSA(prostate specific antigen), AFP(Alpha-fetoprotein), and cTnI (Cardiac Troponin I) and the like. For the lateral flow assay, the value detected is generally corrected for accuracy. For example, Hb may be used for the correction in which case the present integrated device is conveniently used for obtaining both the amount of analyte of interest and the information to be used for the correction. For example, for an HbAlc test, conventionally an A1c test using a lateral flow method and a hemoglobin test using an absorption test are separately performed, which are conveniently performed in one device using the present integrated device. For example, using the integrated device according to the present disclosure, both measurements can be performed at once by using a reader, for example, i-Chroma.

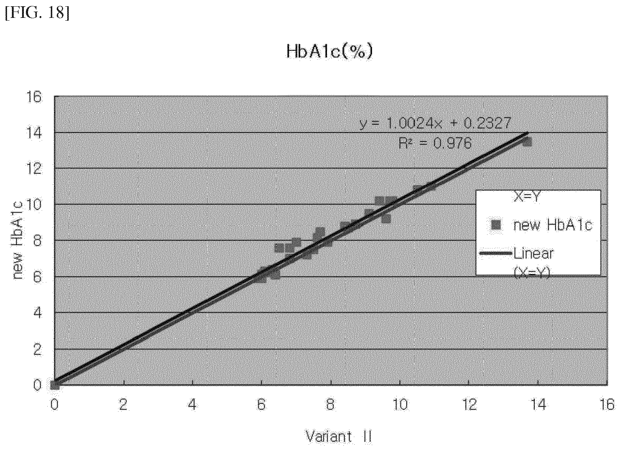

That is, conventionally HsCRP, and A1c information were obtained using lateral flow assay, and absorption was measured separately from the lateral flow assay, which was time consuming and cumbersome. The present integrated device 200 solves such problems by obtaining two information at one measurement. Further, by employing the method measuring the absorbance of the reflected light, the arrangements of the device (200), light source (400) and light detector (500) becomes more flexible and not restricted to a particular arrangement compared to that for measuring the transmitted light. Also as described below and shown in the result described in FIG. 18, the rapid, convenient and accurate assays are secured by using the present device.

In one embodiment, to test the performance of the reflective absorbance measuring device 100 according to the present disclosure and the integrated device 200 for measuring reflective absorbance 210 and lateral flow assay, the amount of Hb was determined. As described above, it is preferred that the light source 400 and the light detector 500 are not longitudinally positioned in a row to minimize the surface reflected light. Therefore, as shown in FIG. 7, the light sources 400 and light detector 500 is horizontally positioned, but the arrangement is not limited thereto.

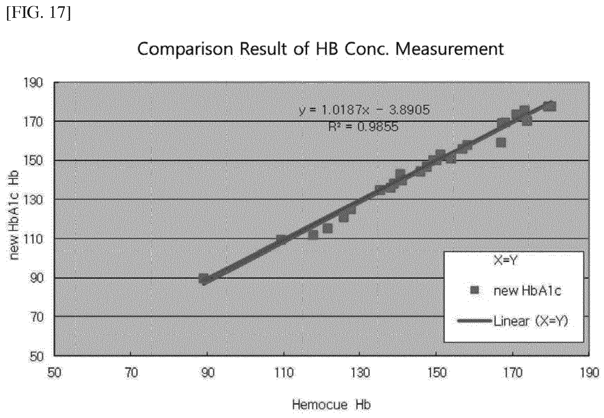

FIG. 17 is a graph comparing the Hb concentration obtained by the present device for measuring reflective absorbance and by the device integrating reflective absorbance measurement and lateral flow assay with the Hb concentration measured using a conventional method, in which the absorbance measured was converted into a concentration of Hb.

In FIG. 17, the system as depicted in FIG. 7 was used, in which the blood sample was applied to the device through the sample inlet 121 and to the sample receiving part 111, and then the light having 520 nm in wavelength was illuminated onto the sample through the light transmitting member 127 from the light source (400)(LED). Then, the light reflected from the sample was detected to obtain the absorbance which was then converted to the Hb concentration.

In FIG. 17, Y axis represents the concentration of Hb obtained by the experiment using the present devices (100, 201), and X axis represents the Hb concentration obtained by Hb-301 by Hemocue Inc. As shown in FIG. 17, Y axis and X axis are analyzed by linear regression as Y = 1.0187X-3.8905 (R2=0. 9855) and they produced substantially the same value (a value close to Y=X).

As can be seen from the results above, the present device can be conveniently used to accurately measure Hb concentration by measuring the reflected light.

Next, to test the performance of the integrated device 200 for measuring reflective absorbance and lateral flow assay, the HbAlc concentration was determined. For this, Hb concentration was determined by measuring the absorbance using the device 210 where part of the sample was applied through the sample inlet 121 to the sample receiving part 111 and the absorbance measured was converted to a concentration. Also Ale was measured by lateral flow assay using the present device 220. Then two values thus obtained were used for the calculation of HbAlc concentration.

FIG. 18 is a graph comparing the HbAlc concentration obtained by the present device for measuring reflective absorbance and by the device integrating reflective absorbance measurement and lateral flow assay with the HbAlc concentration measured using a conventional method, in which the Hb concentration was determined by absorbance, and A1c was determined by lateral flow assay.

That is, FIG.18 is a result comparing the HbAlc concentration, a diabetic marker, obtained by the present integrated device with that obtained by the conventional device, which is a standard device VARIANT II under the quality control.

In FIG. 18, Y axis represents the concentration of HbAlc obtained by the experiment using the present devices (200), and X axis represents the HbAlc concentration obtained by VARIANT II. As shown in FIG. 18, Y axis and X axis was analyzed by linear regression as Y = 1.0024X-0.2327 (R2=0. 976) and they produced substantially the same value (a value close to Y=X).

As can be seen from the test result, the present device can be conveniently used to accurately and rapidly measure the concentration of biological materials combing the information from absorption and lateral flow assay.

### <Description of Reference Numerals>

- 100:: Device for measuring reflective absorbance
- 110:: Base member
- 110a:: Tetragonal portion
- 111:: Sample receiving part
- 111a:: Bottom portion
- 120:: Cover member
- 120a:: Tetragonal hole
- 120b:: Semicircular protrusion
- 121:: Sample inlet
- 123:: Sample inlet
- 125:: Window
- 127:: Light transmitting member
- 127b:: bottom surface of light transmitting member
- 129:: Light blocking member
- 130:: Rim
- 140:: Opening
- 150:: Protrusion
- 200:: Integral device for measuring reflective absorbance and analyzing lateral flow
- 210:: Device for measuring reflective absorbance
- 220:: Device for Lateral flow assay
- 221:: Second sample inlet
- 223:: Measurement window
- 225:: Strip receiving part
- 230:: Rim
- 240:: Cover for measurement window
- 241a:: First guide protrusion
- 241b:: Second guide protrusion
- 243:: Trapping sill
- 245:: Stopping protrusion
- 247:: Receiving recess
- 260:: moving guide
- 261:: First guide groove
- 263:: Second guide groove
- 265:: Rail
- 267:: Third guide groove
- 269:: Fourth guide groove
- 300:: Sample
- 400:: Light source
- 500:: Light detector
- 600:: Strip
- 700:: Housing

## Claims

1. A device (220) for lateral flow assay, the device comprising:
a base member (110) comprising a strip receiving part (225); and
a cover member (120) covering an upper portion of the base member (110) and comprising a sample inlet (221) and a window (223), the window is a structure for measuring a reaction result on the strip, wherein a strip (600) mounted on the strip receiving part (225) so that a sample (300) is introduced through the sample inlet (221) into the mounted strip (600);
**characterized by**
a window cover (240) for opening and closing the window (223), wherein the window cover (240) is positioned being perpendicular to the strip receiving part (225) when the window (223) is closed.

2. The device of Claim 1, wherein the window cover (240) is detachable, and the window cover (240) is opened or closed by sliding.

3. An integrated device (200) for measuring reflective absorbance and lateral flow assay, the integrated device comprising:
one or more device parts (210) for measuring reflective absorbance, wherein each device part (210) for measuring reflective absorbance comprises:
a base member (110) comprising a sample receiving part (111); and
a cover member (120) covering an upper portion of the base member (110);
the cover member (120) comprising a first sample inlet (121, 123) through which a sample (300) is introducible into the sample receiving part (111), and a light transmitting member (127), the light transmitting member (127) being positioned perpendicular to the sample receiving part (111) when the cover member (120) covers the base member (110), wherein the sample receiving part (111) is configured to reflect an incident light illuminated through the light transmitting member (127); the integrated device comprising further:
a device part (220) for lateral flow assay comprising a cover member (120) and a base member (110), wherein the cover member (120) and the base member (110) of the device part (220) for lateral flow assay extend in one direction from the cover member (120) and the base member (110) of the device part (210) for measuring reflective absorbance,
**characterized by** the cover member (120) of the device part (220) for lateral flow assay comprises a second sample inlet (221) and a window (223), the window is a structure for measuring a reaction result on a strip, the base member (110) of the device part (220) for lateral flow assay comprises a strip receiving part (225) for mounting the strip (600) thereon, so that the sample (300) is introduced into the mounted strip (600) through the second sample inlet (221);
wherein the device part (220) for lateral flow assay further comprises a window cover (240), wherein the window cover (240) is detachable, and is able to open or close the window (223) of the device part (220) for lateral flow assay, and is positioned perpendicular to the strip receiving part (225) in a closed state.

4. The integrated device of Claim 3, wherein the second sample inlet (221) of the device part (220) for lateral flow assay is arranged adjacent to the sample inlet (121, 123) formed on the cover member (120) of the device (210) for measuring reflective absorbance.

5. The integrated device of one of Claim 4, wherein the integrated device (200) comprises two or more device parts (210) for measuring reflective absorbance and two or more device parts (220) for lateral flow assay, and the device parts (210) for measuring reflective absorbance and the device parts (220) for lateral flow assay are disposed in a row or side by side, respectively.

6. The integrated device of Claim 3 , wherein the sample receiving part (111) of the device part (210) for measuring reflective absorbance is configured to directly reflect the incident light or further comprises a reflective bottom portion (111a) which it is able to reflect the incident light.

7. The integrated device of Claim 3, wherein the light transmitting member (127) of the device part (210) for measuring reflective absorbance is configured to be inserted into a window (125) formed on the cover member (120) of the device part (210) for measuring reflective absorbance or integrally formed with the cover member (120) of the device part (210) for measuring reflective absorbance, wherein all or part of the cover member (120) of the device part (210) for measuring reflective absorbance other than the light transmitting member (127) is configured to be light-blocked.

8. The integrated device of Claim 7, wherein the light is physically blocked by treating the corresponding cover member (120) with a light blocking material or the light is blocked by using a software.

9. The integrated device of any one of Claims 3 and 6-8, wherein a bottom of the light transmitting member (127) of the device part (210) for measuring reflective absorbance is recessed inward.

10. The integrated device of any one of Claims 3 and 6-9, wherein a protrusion (150) is formed on a bottom of the cover member (120) of the device part (210) for measuring reflective absorbance adjacent to a bottom (127b) of the light transmitting member (127).

11. The integrated device of any one of Claims 3 and 6-10, wherein a distance from a bottom (127b) of the light transmitting member (127) to the sample receiving part (111) of the device part (210) for measuring reflective absorbance is about 0.5 mm to 10 mm.

12. The integrated device of any one of Claims 3 and 6-11, wherein the cover member (120) of the device part (210) for measuring reflective absorbance further comprises an opening (140) in addition to one sample inlet (121, 123), and the opening (140) is located facing the sample inlet (121, 123) of the device part (210) for measuring reflective absorbance with the light transmitting member (127) being disposed therebetween.

13. The integrated device of any one of Claims 3 and 6-12, wherein the cover member (120) of the device part (210) for measuring reflective absorbance comprises a plurality of sample inlets (121, 123).

14. The integrated device of any one of Claims 3 and 6-13, wherein the device part (210) for measuring reflective absorbance has two sample inlets (121, 123), the sample inlets (121, 123) are positioned facing each other with the light transmitting member (127) being disposed therebetween.

15. The integrated device of any one of Claims 3 and 6-14, wherein a tetragonal hole (120a) is formed on the cover member (120) of the device part (210) for measuring reflective absorbance, and a tetragonal portion (110a) is formed at a corresponding position of the base member (110) of the device part (210) for measuring reflective absorbance which is perpendicular to the tetragonal hole (120a) when the cover member (120) and the base member (110) are engaged.

## Patentansprüche

1. Einrichtung (220) zum Lateralfluss-Assay, wobei die Einrichtung Folgendes enthält:
ein Grundelement (110), das einen Streifenaufnahmeteil (225) enthält; und
ein Abdeckelement (120), das einen oberen Teil des Grundelements (110) abdeckt und einen Probeneinlass (221) und ein Fenster (223) enthält, wobei das Fenster ein Aufbau zum Messen eines Reaktionsergebnisses auf dem Streifen ist, wobei
ein Streifen (600) am Streifenaufnahmeteil (225) montiert ist, derart, dass eine Probe (300) durch den Probeneinlass (221) in den montierten Streifen (600) eingeführt wird;
**gekennzeichnet durch**
eine Fensterabdeckung (240), um das Fenster (223) zu öffnen und zu schließen, wobei die Fensterabdeckung (240) senkrecht zum Streifenaufnahmeteil (225) positioniert ist, wenn das Fenster (223) geschlossen ist.

2. Einrichtung nach Anspruch 1, wobei die Fensterabdeckung (240) abnehmbar ist und die Fensterabdeckung (240) durch Verschieben geöffnet oder geschlossen wird.

3. Integrierte Einrichtung (200) zum Messen der reflektierenden Absorbanz und des Lateralfluss-Assays, wobei die integrierte Einrichtung Folgendes enthält:
ein oder mehrere Einrichtungsteile (210) zum Messen der reflektierenden Absorbanz, wobei jeder Einrichtungsteil (210) zum Messen der reflektierenden Absorbanz Folgendes enthält:
ein Grundelement (110), das einen Probenaufnahmeteil (111) enthält; und
ein Abdeckelement (120), das einen oberen Teil des Grundelements (110) abdeckt; wobei
das Abdeckelement (120) einen ersten Probeneinlass (121, 123), durch den eine Probe (300) in den Probenaufnahmeteil (111) eingesetzt werden kann, und ein lichtübertragendes Element (127) enthält, wobei das lichtübertragende Element (127) senkrecht zum Probenaufnahmeteil (111) positioniert ist, wenn das Abdeckelement (120) das Grund-element (110) abdeckt, wobei der Probenaufnahmeteil (111) konfiguriert ist, einfallendes Licht, das durch das lichtübertragende Element (127) eingestrahlt wird, zu reflektieren; wobei
die integrierte Einrichtung ferner Folgendes enthält:
einen Einrichtungsteil (220) zum Lateralfluss-Assay, der ein Abdeckelement (120) und ein Grundelement (110) umfasst, wobei sich das Abdeckelement (120) und das Grundelement (110) des Einrichtungsteils (220) zum Lateralfluss-Assay in einer Richtung vom Abdeckelement (120) und vom Grundelement (110) des Einrichtungsteils (210) zum Messen der reflektierenden Absorbanz erstrecken,
**dadurch gekennzeichnet, dass**
das Abdeckelement (120) des Einrichtungsteils (220) zum Lateralfluss-Assay einen zweiten Probeneinlass (221) und ein Fenster (223) enthält, wobei das Fenster ein Aufbau zum Messen eines Reaktionsergebnisses auf dem Streifen ist,
das Grundelement (110) des Einrichtungsteils (220) zum Lateralfluss-Assay einen Streifenaufnahmeteil (225) enthält, um den Streifen (600) an ihm zu montieren, derart, dass die Probe (300) durch den Probeneinlass (221) in den montierten Streifen (600) eingeführt wird; wobei
der Einrichtungsteil (220) zum Lateralfluss-Assay ferner eine Fensterabdeckung (240) enthält, wobei die Fensterabdeckung (240) abnehmbar ist, das Fenster (223) des Einrichtungsteils (220) zum Lateralfluss-Assay öffnen und schließen kann und in einem geschlossenen Zustand senkrecht zum Streifenaufnahmeteil (225) positioniert ist.

4. Integrierte Einrichtung nach Anspruch 3, wobei der zweite Probeneinlass (221) des Einrichtungsteils (220) zum Lateralfluss-Assay angrenzend an den Probeneinlass (121, 123), der am Abdeckelement (120) der Einrichtung (210) zum Messen der reflektierenden Absorbanz gebildet ist, angeordnet ist.

5. Integrierte Einrichtung nach Anspruch 4, wobei die integrierte Einrichtung (200) zwei oder mehr Einrichtungsteile (210) zum Messen der reflektierenden Absorbanz und zwei oder mehr Einrichtungsteile (220) zum Lateralfluss-Assay umfasst und die Einrichtungsteile (210) zum Messen der reflektierenden Absorbanz und die Einrichtungsteile (220) zum Lateralfluss-Assay jeweils in einer Reihe oder nebeneinander angeordnet sind.

6. Integrierte Einrichtung nach Anspruch 3, wobei der Probenaufnahmeteil (111) des Einrichtungsteils (210) zum Messen der reflektierenden Absorbanz konfiguriert ist, das einfallende Licht direkt zu reflektieren oder ferner einen reflektierenden Unterseitenteil (111a), der das einfallende Licht reflektieren kann, enthält.

7. Integrierte Einrichtung nach Anspruch 3, wobei das lichtübertragende Element (127) des Einrichtungsteils (210) zum Messen der reflektierenden Absorbanz konfiguriert ist, in ein Fenster (125), das am Abdeckelement (120) des Einrichtungsteils (210) zum Messen der reflektierenden Absorbanz gebildet ist, oder das einteilig mit dem Abdeckelement (120) des Einrichtungsteils (210) zum Messen der reflektierenden Absorbanz gebildet ist, eingesetzt zu sein, wobei das gesamte Abdeckelement (120) des Einrichtungsteils (210) oder ein Teil davon zum Messen der reflektierenden Absorbanz mit Ausnahme des lichtübertragenden Elements (127) konfiguriert ist, lichtblockiert zu sein.

8. Integrierte Einrichtung nach Anspruch 7, wobei das Licht physikalisch durch Behandeln des entsprechenden Abdeckelements (120) mit einem lichtsperrenden Material blockiert wird oder das Licht unter Verwendung einer Software blockiert wird.

9. Integrierte Einrichtung nach einem der Ansprüche 3 und 6-8, wobei eine Unterseite des lichtübertragenden Elements (127) des Einrichtungsteils (210) zum Messen der reflektierenden Absorbanz nach innen versenkt ist.

10. Integrierte Einrichtung nach einem der Ansprüche 3 und 6-9, wobei ein Vorsprung (150) an einer Unterseite des Abdeckelements (120) des Einrichtungsteils (210) zum Messen der reflektierenden Absorbanz angrenzend an eine Unterseite (127b) des lichtübertragenden Elements (127) gebildet ist.

11. Integrierte Einrichtung nach einem der Ansprüche 3 und 6-10, wobei eine Entfernung von einer Unterseite (127b) des lichtübertragenden Elements (127) zum Probenaufnahmeteil (111) des Einrichtungsteils (210) zum Messen der reflektierenden Absorbanz im Bereich von etwa 0,5 mm bis 10 mm liegt.

12. Integrierte Einrichtung nach einem der Ansprüche 3 und 6-11, wobei das Abdeckelement (120) des Einrichtungsteils (210) zum Messen der reflektierenden Absorbanz ferner zusätzlich zu einem Probeneinlass (121, 123) eine Öffnung (140) enthält und die Öffnung (140) dem Probeneinlass (121, 123) des Einrichtungsteils (210) zum Messen der reflektierenden Absorbanz zugewandt angeordnet ist, wobei das lichtübertragende Element (127) dazwischen angeordnet ist.

13. Integrierte Einrichtung nach einem der Ansprüche 3 und 6-12, wobei das Abdeckelement (120) des Einrichtungsteils (210) zum Messen der reflektierenden Absorbanz mehrere Probeneinlässe (121, 123) enthält.

14. Integrierte Einrichtung nach einem der Ansprüche 3 und 6-13, wobei der Einrichtungsteil (210) zum Messen der reflektierenden Absorbanz zwei Probeneinlässe (121, 123) besitzt, wobei die Probeneinlässe (121, 123) einander zugewandt positioniert sind, wobei das lichtübertragende Element (127) dazwischen angeordnet ist.

15. Integrierte Einrichtung nach einem der Ansprüche 3 und 6-14, wobei ein tetragonales Loch (120a) am Abdeckelement (120) des Einrichtungsteils (210) zum Messen der reflektierenden Absorbanz gebildet ist und ein tetragonaler Teil (110a) bei einem entsprechenden Teil des Grundelements (110) des Einrichtungsteils (210) zum Messen der reflektierenden Absorbanz, der senkrecht zum tetragonalen Loch (120a) ist, wenn das Abdeckelement (120) und das Grundelement (110) in Eingriff sind, gebildet ist.

## Revendications

1. Dispositif (220) pour test à écoulement latéral, le dispositif comprenant :
un élément de base (110) comprenant une partie de réception de bandelette (225) ; et
un élément de recouvrement (120) recouvrant une partie supérieure de l'élément de base (110) et comprenant une entrée d'échantillon (221) et une fenêtre (223), la fenêtre est une structure pour mesurer un résultat de réaction sur la bandelette,
dans lequel une bandelette (600) est montée sur la partie de réception de bandelette (225) de sorte qu'un échantillon (300) est introduit à travers l'ouverture d'échantillon (221) dans la bandelette (600) montée ;
**caractérisé par** un couvercle de fenêtre (240) pour ouvrir et fermer la fenêtre (223), dans lequel le couvercle de fenêtre (240) est positionné en étant perpendiculaire à la partie de réception de bandelette (225) lorsque la fenêtre (223) est fermée.

2. Dispositif selon la revendication 1, dans lequel le couvercle de fenêtre (240) est détachable, et le couvercle de fenêtre (240) est ouvert ou fermé par coulissement.

3. Dispositif intégré (200) pour mesurer l'absorbance réfléchissante et pour le test d'écoulement latéral, le dispositif intégré comprenant :
une ou plusieurs parties de dispositif (210) pour mesurer l'absorbance réfléchissante, dans lequel chaque partie de dispositif (210) pour mesurer l'absorbance réfléchissante comprend :
un élément de base (110) comprenant une partie de réception d'échantillon (111) ; et
un élément de recouvrement (120) recouvrant une partie supérieure de l'élément de base (110) ;
l'élément de recouvrement (120) comprenant une première entrée d'échantillon (121, 123) à travers laquelle un échantillon (300) peut être introduit dans la partie de réception d'échantillon (111), et un élément de transmission de lumière (127), l'élément de transmission de lumière (127) étant positionné perpendiculairement à la partie de réception d'échantillon (111) lorsque l'élément de recouvrement (120) recouvre l'élément de base (110), dans lequel la partie de réception d'échantillon (111) est configurée pour réfléchir une lumière incidente qui s'illumine via l'élément de transmission de lumière (127) ;
le dispositif intégré comprenant en outre :
une partie de dispositif (220) pour le test d'écoulement latéral comprenant un élément de recouvrement (120) et un élément de base (110), dans lequel l'élément de recouvrement (120) et l'élément de base (110) de la partie de dispositif (220) pour le test d'écoulement latéral s'étendent dans une direction à partir de l'élément de recouvrement (120) et l'élément de base (110) de la partie de dispositif (210) pour mesurer l'absorbance réfléchissante,
**caractérisé en ce que** l'élément de recouvrement (120) de la partie de dispositif (220) pour le test d'écoulement latéral comprend une deuxième entrée d'échantillon (221) et une fenêtre (223), la fenêtre est une structure pour mesurer un résultat de réaction sur une bandelette, l'élément de base (110) de la partie de dispositif (220) pour le test d'écoulement latéral comprend une partie de réception de bandelette (225) pour monter la bandelette (600) sur celle-ci, de sorte que l'échantillon (300) est introduit à travers la deuxième ouverture d'échantillon (221) dans la bandelette (600) montée ;
dans lequel la partie de dispositif (220) pour le test d'écoulement latéral comprend en outre un couvercle de fenêtre (240), dans lequel le couvercle de fenêtre (240) est détachable et peut ouvrir ou fermer la fenêtre (223) de la partie de dispositif (220) pour le test d'écoulement latéral et est positionné perpendiculairement à la partie de réception de bandelette (225) dans un état fermé.

4. Dispositif intégré selon la revendication 3, dans lequel la deuxième entrée d'échantillon (221) de la partie de dispositif (220) pour le test d'écoulement latéral est disposée de façon adjacente à l'entrée d'échantillon (121, 123) formée sur l'élément de recouvrement (120) du dispositif (210) pour mesurer l'absorbance réfléchissante.

5. Dispositif intégré selon l'une de la revendication 4, dans lequel le dispositif intégré (200) comprend deux ou davantage de parties de dispositif (210) pour mesurer l'absorbance réfléchissante et deux ou davantage de parties de dispositif (220) pour le test d'écoulement latéral, et les parties de dispositif (210) pour mesurer l'absorbance réfléchissante et les parties de dispositif (220) pour le test d'écoulement latéral sont respectivement disposées sur une rangée ou côte à côte.

6. Dispositif intégré selon la revendication 3, dans lequel la partie de réception d'échantillon (111) de la partie de dispositif (210) pour mesurer l'absorbance réfléchissante est configurée pour directement réfléchir la lumière incidente ou comprend en outre une partie inférieure réfléchissante (111a) capable de réfléchir la lumière incidente.

7. Dispositif intégré selon la revendication 3, dans lequel l'élément de transmission de lumière (127) de la partie de dispositif (210) pour mesurer l'absorbance réfléchissante est configuré pour être inséré dans une fenêtre (125) formée sur l'élément de recouvrement (120) de la partie de dispositif (210) pour mesurer l'absorbance réfléchissante ou est formé d'un seul tenant avec l'élément de recouvrement (120) de la partie de dispositif (210) pour mesurer l'absorbance réfléchissante, dans lequel la totalité ou une partie de l'élément de recouvrement (120) de la partie de dispositif (210) pour mesurer l'absorbance réfléchissante autre que l'élément de transmission de lumière (127) est configurée pour être à lumière bloquée.

8. Dispositif intégré selon la revendication 7, dans lequel la lumière est bloquée physiquement en traitant l'élément de recouvrement (120) correspondant avec une matière bloquant la lumière, ou bien la lumière est bloquée en utilisant un logiciel.

9. Dispositif intégré selon l'une quelconque des revendications 3 et 6-8,
dans lequel une partie inférieure de l'élément de transmission de lumière (127) de la partie de dispositif (210) pour mesurer l'absorbance réfléchissante est creusée vers l'intérieur.

10. Dispositif intégré selon l'une quelconque des revendications 3 et 6-9,
dans lequel une saillie (150) est formée sur une partie inférieure de l'élément de recouvrement (120) de la partie de dispositif (210) pour mesurer l'absorbance réfléchissante de façon adjacente à une partie inférieure (127b) de l'élément de transmission de lumière (127).

11. Dispositif intégré selon l'une quelconque des revendications 3 et 6-10,
dans lequel une distance entre une partie inférieure (127b) de l'élément de transmission de lumière (127) et la partie de réception d'échantillon (111) de la partie de dispositif (210) pour mesurer l'absorbance réfléchissante est de près de 0,5 mm à 10 mm.

12. Dispositif intégré selon l'une quelconque des revendications 3 et 6-11,
dans lequel l'élément de recouvrement (120) de la partie de dispositif (210) pour mesurer l'absorbance réfléchissante comprend en outre une ouverture (140) en plus d'une entrée d'échantillon (121, 123), et l'ouverture (140) est située de manière à faire face à l'entrée d'échantillon (121, 123) de la partie de dispositif (210) pour mesurer l'absorbance réfléchissante, avec l'élément de transmission de lumière (127) disposé entre elles.

13. Dispositif intégré selon l'une quelconque des revendications 3 et 6-12,
dans lequel l'élément de recouvrement (120) de la partie de dispositif (210) pour mesurer l'absorbance réfléchissante comprend une pluralité d'entrées d'échantillon (121, 123).

14. Dispositif intégré selon l'une quelconque des revendications 3 et 6-13,
dans lequel la partie de dispositif (210) pour mesurer l'absorbance réfléchissante a deux entrées d'échantillon (121, 123), les entrées d'échantillon (121, 123) sont positionnées en se faisant face l'une l'autre avec l'élément de transmission de lumière (127) disposé entre elles.

15. Dispositif intégré selon l'une quelconque des revendications 3 et 6-14,
dans lequel un trou quadratique (120a) est formé sur l'élément de recouvrement (120) de la partie de dispositif (210) pour mesurer l'absorbance réfléchissante, et une partie quadratique (110a) est formée dans une position correspondante de l'élément de base (110) de la partie de dispositif (210) pour mesurer l'absorbance réfléchissante qui est perpendiculaire au trou quadratique (120a) lorsque l'élément de recouvrement (120) et l'élément de base (110) sont en prise.
